# EUROPEAN PATENT SPECIFICATION

(11) **EP 3 489 362 B1**
(45) Date of publication and mention of the grant of the patent: **11.09.2024**
(21) Application number: 18214778.5
(22) Date of filing: 19.10.2015
(51) Int. Cl.: C12N 15/85, A61P 35/00

(54) **BIPARTITE AND TRIPARTITE SIGNALING IMMUNE CELLS**
ZWEITEILIGE UND DREITEILIGE SIGNALISIERENDE IMMUNZELLEN
CELLULES IMMUNITAIRES BIPARTITES ET TRIPARTITES DE SIGNALISATION

(30) Priority: 17.10.2014 US 201462065138 P; 22.04.2015 US 201562151315 P
(43) Date of publication of application: 29.05.2019
(62) Divisional of application: 15850940.6
(73) Proprietor: Baylor College of Medicine, Houston, TX 77030-3411 (US)
(72) Inventor: VALDES, Juan Fernando Vera, Bellaire, TX 77401 (US); LEEN, Ann Marie, Bellaire, TX 77401 (US); SUKUMARAN, Sujita, Houston, TX 77019 (US)
(74) Representative: J A Kemp LLP

(56) References cited:
- WO-A1-2012/138858
- WO-A1-2014/055657
- WO-A1-2014/172584
- US-A1- 2014 120 622
- E. LANITIS ET AL: "Chimeric Antigen Receptor T Cells with Dissociated Signaling Domains Exhibit Focused Antitumor Activity with Reduced Potential for Toxicity In Vivo", CANCER IMMUNOLOGY RESEARCH, vol. 1, no. 1, 7 April 2013 (2013-04-07), pages 43 - 53, XP055170367, ISSN: 2326-6066, DOI: 10.1158/2326-6066.CIR-13-0008
- ALVAREZ-VALLINA L ET AL: "ANTIGEN-SPECIFIC TARGETING OF CD28-MEDIATED T CELL CO-STIMULATION USING CHIMERIC SINGLE-CHAIN ANTIBODY VARIABLE FRAGMENT-CD28 RECEPTORS", EUROPEAN JOURNAL OF IMMUNOLOGY,, vol. 26, no. 10, 1 October 1996 (1996-10-01), pages 2304 - 2309, XP000646504, ISSN: 0014-2980, DOI: 10.1002/EJI.1830261006
- MARC CARTELLIERI ET AL: "Chimeric Antigen Receptor-Engineered T Cells for Immunotherapy of Cancer", JOURNAL OF BIOMEDICINE AND BIOTECHNOLOGY, vol. 21, no. 4, 1 January 2010 (2010-01-01), pages 427 - 13, XP055206629, ISSN: 1110-7243, DOI: 10.1155/2010/956304
- ANN M LEEN ET AL: "Reversal of Tumor Immune Inhibition Using a Chimeric Cytokine Receptor", MOLECULAR THERAPY, & 15TH ANNUAL MEETING OF THE AMERICAN-SOCIETY-OF-GENE-AND-CELL-THERAPY (ASGCT); PHILADELPHIA, PA, USA; MAY 16 -19, 2012, vol. 22, no. 6, 1 June 2014 (2014-06-01), pages 1211 - 1220, XP002728753, ISSN: 1525-0016, DOI: 10.1038/MT.2014.47

## Description

### TECHNICAL FIELD

Embodiments of the disclosure concerns at least the fields of immunology, immunotherapy, cell biology, molecular biology, and medicine, including cancer medicine.

### BACKGROUND

T cell specificity can be altered by expressing chimeric antigen receptors (CAR), which are artificial receptors composed of an extracellular domain that is responsible for antigen recognition, a transmembrane domain, and one or more intracellular signaling domains. The extracellular domain is most commonly derived from the variable regions (i.e. antigen binding portion) of the heavy and light chains (V_{H} and V_{L},) of a monoclonal antibody joined by a flexible linker. The intracellular signaling domain (endodomain) is most usually derived from the T cell receptor (CD3) ζ chain. CAR expression allows tumors to be targeted in an HLA-unrestricted manner, increasing the number of eligible patients, and extends the types of antigens that can be recognized by T cells to include carbohydrates and glycolipids. However initial trials using T cells modified to express CARs that contained exclusively the CD3ζ signaling domain (so called first generation constructs) have proved to be sub-optimal. Indeed, CAR engagement failed to induce either cytokine production or T cell expansion *in vivo.* To overcome this limitation, scientists have explored the use of second and third generation CARs that incorporate additional endodomains, including, for example, CD27, CD28, 4-1BB, DAP10, OX40 or ICOS. Such additions conferred greater strength of signaling and persistence to the T cells, resulting in improved potency. For example, in a head-to-head comparison, Savoldo and colleagues demonstrated that CAR-CD19 T cells encoding the costimulatory CD28 endodomain had strikingly enhanced expansion and persistence. Porter and colleagues used CAR-CD19-modified T cells expressing the 4-1BB endodomain to treat chronic lymphocytic leukemia (CLL) and observed significant *in vivo* expansion, and persistence for at least 6 months, which resulted in complete clinical responses in 2 of 3 treated patients.

Consequently, second and third generation CARs that incorporate additional endodomains has become the current state of the art technology in CAR therapy to improve T cell potency. However, some patients treated with the later generation CARs have experienced toxicities associated with the therapy due to cytokine storm because of uncontrolled expansion of the T cells. The present disclosure provides a solution for such challenges in immunotherapy.

WO 2014/055657 discloses a trans-signaling CAR T cells that comprise a first CAR having a first signaling module and a second AR having a distinct second signaling module. Lanitis et al. (Cancer Immunology Research, 2013, vol. 1, no. 1, pages 43 - 45) discloses that chimeric antigen receptor T cells with dissociated signaling domains exhibit focused anti-tumor activity with reduced potential for toxicity in vivo. Alvarez-Vallina et al. ( European Journal Of Immunology, 1996, vol. 26, no. 10, pages 2304 - 2309) discloses antigen-specific targeting of CD28-mediated T cell co-stimulation using chimeric single-chain antibody variable fragment-C28 receptors. US 2014/0120622 discloses methods and compositions for modifying T-cells in which PD1 and/or CTLA-4 is repressed and/or inactivated using fusion proteins such as artificial transcription factors and nucleases. Leen et al. ("Molecular Therapy, 2012, vol. 22, no. 6, pages 1211 - 1220) discloses reversal of tumor immune inhibition using a chimeric cytokine receptor.

### BRIEF SUMMARY OF THE DISCLOSURE

Described herein are compositions and methods for treatment of a medical condition that requires immunotherapy, including immunotherapy for cancer, for example. In particular aspects of the disclosure, there are compositions and methods that employ immune cells that have been modified to express two or more separate molecules (such as polypeptides) that transmit signals (and which may be separate signals) to activate the immune cells. The signals originate directly or indirectly from a tumor or tumor microenvironment, in particular embodiments.

In certain aspects described herein, a non-natural cell is provided, and in specific embodiments, the cell expresses moieties that receive signals for T cell activation, such as for killing; for co-stimulation, such as for persistence of the cell; and for cytokines, such as to enhance proliferation. In specific aspects described herein, the three signals to the cell result from three different inputs from a microenvironment and are transmitted through three separate molecules, such as three separate receptors, for example. In particular aspects of the disclosure in, the cells contemplated herein are used for cancer therapy in the presence of the signals, and in specific aspects of the disclosure therapy does not occur in the absence of any of the signals.

In some aspects described herein for illustrative purposes, the immune cells are bipartite signaling immune cells that convey two signals from the tumor or tumor microenvironment, wherein each of the two signals are transmitted through separate molecules. In specific aspects of the bipartite signaling immune cells, a first signal is from an antigen (such as a tumor antigen) and is recognized by a molecule (such as a receptor) that binds the antigen. In certain aspects, the binding of the first signal, the antigen, leads to T cell receptor (TCR) activation. In at least some cases, the TCR activation occurs by having the receptor linked to an intracellular co-stimulatory domain that does not directly receive a signal. In particular aspects, a second signal of the bipartite signaling immune cells is from one or more cytokines and is received *via* a molecule (that is separate from the antigen recognition molecule) that transmits the cytokine signal. Exemplary second signaling molecules for the bipartite signaling immune cells may be a cytokine receptor. In specific aspects, the cytokine receptor is a chimeric cytokine receptor that binds inhibitory/suppressive cytokines yet comprises an endodomain that is capable of an activation signal; in particular aspects, the chimeric cytokine receptor comprises a cytokine-binding exodomain and a signal transducing endodomain. Thus, in specific aspects, a bipartite signaling immune cell comprises a first molecule that recognizes an antigen and a second molecule, separate from the first molecule, that recognizes a soluble factor, such as a cytokine.

Also described herein are immune cells that are tripartite signaling immune cells that convey three signals from the tumor or tumor microenvironment, wherein each of the three signals are transmitted through separate molecules. In specific aspects of the tripartite signaling immune cells, a first signal is from an antigen (such as a tumor antigen) and is recognized by a molecule (such as a receptor) that binds the antigen. A second signal is for co-stimulation and is recognized by a molecule (such as a receptor) that binds a soluble factor (such as a co-stimulatory signal), in certain aspects. A third signal, in particular aspects, is a cytokine that is recognized by a molecule (such as a receptor) that binds the soluble factor (a cytokine, for example) and transmits a positive signal for proliferation. Thus, in specific aspects, a tripartite signaling immune cell comprises a first molecule that recognizes an antigen, a second molecule, separate from the first molecule, that recognizes a soluble factor (including a co-stimulatory molecule, for example), and a third molecule, separate from both the first and second molecules, that recognizes a soluble factor, such as a cytokine.

In one aspect described herein, a composition comprises an engineered immune cell that separately expresses 1) an antigen receptor; 2) a cytokine receptor; and 3) a co-stimulation receptor. In particular aspects of the disclosure, the immune cells are tailored by design to be specific for an antigen from a particular tumor or tumor microenvironment. For example, as set out below, the invention provides a composition, comprising an engineered T cell that separately expresses: 1) a chimeric antigen receptor (CAR) that provides antigen signaling for the cell upon binding of PSCA; 2) a co-stimulation receptor that provides co-stimulation for the cell, which is a chimeric cytokine receptor comprising a TGFβR exodomain and a 4-1BB endodomain; and 3) a cytokine receptor that provides cytokine stimulation for the cell, which is a chimeric cytokine receptor comprising an IL-4 receptor exodomain and an IL-7 receptor endodomain; wherein the antigen receptor and the co-stimulation receptor are different molecules.

In one aspect described herein for illustrative purposes, a composition comprises an engineered immune cell that separately expresses at least two of the following: 1) a molecule that provides antigen recognition for the cell; 2) a molecule that provides a co-stimulatory signal for the cell; and 3) a molecule that provides cytokine stimulation for the cell, wherein there is one of the following a) the molecule that provides antigen recognition for the cell and the molecule that provides co-stimulation for the cell are the same molecule; or b) the molecule that provides antigen recognition for the cell and the molecule that provides a co-stimulatory signal for the cell are different molecules.

In some aspects described herein for illustrative purposes, there is a composition, comprising an engineered immune cell that separately expresses at least two of: a) a molecule that provides co-stimulatory signaling to the cell upon recognition of a first soluble factor; b) a molecule that provides cytokine signaling to the cell upon recognition of a second soluble factor; and c) a molecule that provides antigen recognition for the cell upon recognition of a soluble antigen (although in alternative cases the antigen is on the surface of a cell), wherein the molecule that provides antigen recognition for the cell is native to the cell or artificial to the cell, wherein when the molecule that provides antigen recognition for the cell is an artificial receptor, the artificial receptor lacks a co-stimulatory domain. In specific aspects, when the molecule that provides antigen recognition for the cell is an artificial receptor that is a chimeric antigen receptor, the chimeric antigen receptor lacks a co-stimulatory domain. In particular aspects, molecule a) and/or molecule b) comprises a single endodomain. In specific aspects, the first soluble factor and the second soluble factor are non-identical. In particular aspects, the cell separately expresses a), b), and c); or, the cell separately expresses a) and c); or, the cell separately expresses b) and c).

The cancer treated by methods and compositions contemplated herein may be of any kind, but exemplary cancers include, but are not limited to, prostate, breast, melanoma, pancreatic, lung, brain, colon, esophageal, liver, kidney, testicular, ovarian, cervical, gall bladder, thyroid, anal, endometrial, bladder, pituitary gland, leukemia, lymphoma, stomach, and spleen.

In aspects described herein for illustrative purposes, there is a composition, comprising an engineered immune cell that separately expresses at least two of: 1) a molecule that provides antigen recognition for the cell; 2) a molecule that provides co-stimulation for the cell; and 3) a molecule that provides cytokine stimulation for the cell, wherein there is one of the following: a) the molecule that provides antigen recognition for the cell and the molecule that provides co-stimulation for the cell are the same molecule; or b) the molecule that provides antigen recognition for the cell and the molecule that provides co-stimulation for the cell are different molecules. In specific aspects of the cell compositions of the disclosure, the molecule that provides antigen recognition is a receptor, the molecule that provides cytokine stimulation is a receptor, or both. In certain aspects of the composition, the molecule that provides co-stimulation for the cell is an endodomain. In some aspects of the composition, a transmembrane domain is positioned in the molecule between the molecule that provides antigen recognition for the cell and the molecule that provides co-stimulation for the cell. In particular aspects of the composition, the antigen is a tumor antigen. In certain aspects, the antigen is a tumor antigen selected from the group that includes but is not limited to EphA2, HER2, GD2, Glypican-3, 5T4, 8H9, αvβ6 integrin, B cell maturation antigen (BCMA), B7-H3, B7-H6, CAIX, CA9, CD19, CD20, CD22, kappa light chain, CD30, CD33, CD38, CD44, CD44v6, CD44v7/8, CD70, CD123, CD138, CD171, CEA, CSPG4, EGFR, EGFRvIII, EGP2, EGP40, EPCAM, ERBB3, ERBB4, ErbB3/4, FAP, FAR, FBP, fetal AchR, Folate Receptor α, GD2, GD3, HLA-AI MAGE A1, HLA-A2, IL11Ra, IL13Ra2, KDR, Lambda, Lewis-Y, MCSP, Mesothelin, Muc1, Muc16, NCAM, NKG2D ligands, NY-ESO-1, PRAME, PSCA, PSC1, PSMA, ROR1, Sp17, SURVIVIN, TAG72, TEM1, TEM8, VEGRR2, carcinoembryonic antigen, HMW-MAA, and VEGF receptor.

In certain aspects the cell compositions of the disclosure, the molecule that provides antigen recognition and the molecule that provides co-stimulation is a chimeric antigen receptor, and the chimeric antigen receptor comprises one or two co-stimulatory endodomains. In particular aspects of the composition, the molecule that provides antigen recognition is a recombinantly produced αβT-cell receptor (TCR) or a native αβTCR. In certain aspects of the composition, the molecule that provides cytokine stimulation is a cytokine receptor, and the cytokine receptor may be a chimeric cytokine receptor comprising a cytokine-binding exodomain and a signal transducing endodomain. In specific aspects, the chimeric cytokine receptor has an endodomain that can be derived from receptors/molecules including TLR1,TLR2, TLR3, TLR4, TLR5, TLR6, TLR7, TLR8, TLR9, CD28, OX-40, 4-1BB, CD80, CD86, ICOS, CD40, CD27, CD30, CD226, IL7, IL2, IL15, IL21, IL12, IL18, IL9, and IFN-gamma that can transmit stimulatory signals (signal 2 (persistence) or signal 3 (cytokine release) or both). In particular aspects, the chimeric cytokine receptor has an exodomain derived from receptors/molecules that can bind to soluble inhibitory factors including TGFβ, IL10, IL4, IL13, IL6, IL8, IL5, VEGF, IL22, IL1, IL1β, IL35, TNF, GM-CSF, M-CSF, G-CSF, and chemokine receptors. In a specific aspect, the chimeric cytokine receptor has an IL4 exodomain and an IL7 endodomain. In a specific aspects, the chimeric cytokine receptor has a TGFβR exodomain and a 4-1BB endodomain.

In particular aspects of the cell compositions of the disclosure, the molecule that provides antigen recognition and co-stimulation for the cell and the molecule that provides cytokine stimulation for the cell are expressed from the same expression vector. In certain aspects of the composition, the molecule that provides antigen recognition and co-stimulation for the cell and the molecule that provides cytokine stimulation for the cell are expressed from a different expression vector. In particular aspects, the molecule that provides antigen recognition, the molecule that provides co-stimulation, and the molecule that provides cytokine stimulation are all expressed from the same expression vector. In specific aspects of the composition, the molecule that provides antigen recognition, the molecule that provides co-stimulation, and the molecule that provides cytokine stimulation are all expressed from different expression vectors. Vectors include viral vectors or non-viral vectors. Viral vectors may be a retroviral vector, lentiviral vector, adenoviral vector, or adeno-associated viral vector.

Any composition of the disclosure may be any type of cell, including any type of immune cell, such as a T cell, a natural killer (NK) cell, or a NKT cell. In specific aspects of the disclosure, the cell further comprises a naturally occurring or engineered T cell receptor that targets a tumor antigen that is the same antigen of the composition. In particular aspects of the disclosure, the cell further comprises a naturally occurring or engineered T cell receptor that targets a tumor antigen that is a different antigen from the antigen of the composition.

In one aspect described herein for illustrative purposes, there is a composition, comprising an engineered immune cell that separately expresses at least two of: a) a molecule that provides costimulatory signaling to the cell upon recognition of a first soluble factor; b) a molecule that provides cytokine signaling to the cell upon recognition of a second soluble factor; and c) a molecule that provides antigen recognition for the cell upon recognition of a antigen, wherein the molecule is native to the cell or artificial to the cell, wherein when the molecule that provides antigen recognition for the cell is an artificial receptor that is a chimeric antigen receptor, the chimeric antigen receptor lacks a costimulatory domain. In specific aspects of the composition, molecule a) comprises a single endodomain. In specific aspects of the composition, molecule b) comprises a single endodomain. In particular aspects, the first soluble factor and the second soluble factor are non-identical. In certain aspects, the cell separately expresses a), b), and c). The cell may separately express a) and c). The cell may separately express b) and c). In a specific aspect, the molecule that provides cytokine signaling is a chimeric cytokine receptor comprising a cytokine-binding exodomain and a signal transducing endodomain.

In one aspect disclosed herein for illustrative purposes, there is a composition, comprising an engineered immune cell that separately expresses at least two of: a) a molecule that provides costimulatory signaling to the cell upon recognition of a first soluble factor; b) a molecule that provides cytokine signaling to the cell upon recognition of a second soluble factor; and c) a molecule that provides antigen recognition for the cell upon recognition of an antigen, wherein the molecule is native to the cell or artificial to the cell, wherein when the molecule that provides antigen recognition for the cell is an artificial receptor, the artificial receptor lacks a costimulatory domain.

One aspect disclosed herein provides a composition encompassed by the disclosure, for use in a method of treating an individual in need of immunotherapy for a medical condition, the method comprising the step of delivering a therapeutically effective amount of the composition encompassed to the individual. In specific aspects, the medical condition is cancer, and the cancer may have a tumor microenvironment comprising the antigen and soluble factors, the levels of which are sufficient to activate the cells through all of the molecules 1), 2), and 3) (where 1) a molecule that provides antigen recognition for the cell; 2) a molecule that provides co-stimulation for the cell; and 3) a molecule that provides cytokine stimulation for the cell).

In one aspect of the disclosure, there is a kit comprising any composition encompassed by the disclosure, said composition housed in a suitable container. Any references in the description to methods of treatment refer to the compounds, pharmaceutical compositions and medicaments of the present invention for use in a method for treatment of the human (or animal) body by therapy (or for diagnosis).

### SUMMARY OF THE INVENTION

The invention provides a composition, comprising an engineered T cell that separately expresses: 1) a chimeric antigen receptor (CAR) that provides antigen signaling for the cell upon binding of PSCA; 2) a co-stimulation receptor that provides co-stimulation for the cell, which is a chimeric cytokine receptor comprising a TGFβR exodomain and a 4-1BB endodomain; and 3) a cytokine receptor that provides cytokine stimulation for the cell, which is a chimeric cytokine receptor comprising an IL-4 receptor exodomain and an IL-7 receptor endodomain; wherein the antigen receptor and the co-stimulation receptor are different molecules.

The invention also provides:
- the composition of the invention, for use in a method of treating an individual in need of immunotherapy for a medical condition, comprising the step of delivering a therapeutically effective amount of the composition to the individual; and
- a kit comprising the composition of the invention, said composition housed in a suitable container

### BRIEF DESCRIPTION OF THE DRAWINGS

For a more complete understanding of the present disclosure, reference is now made to the following descriptions taken in conjunction with the accompanying drawings.
FIG. 1 shows an example of a third generation CAR from prior art that incorporates additional endodomains to improve T cell potency wherein the sole presence of the antigen results in the activation of the three different signal pathways of T cell activation, co-stimulation, and cytokine signaling;
FIG. 2 illustrates an aspect of the disclosure wherein signals I (antigen stimulation (a)), II (co-stimulation (b)) and III (cytokine signal (c)) are separated into 3 different receptors, allowing the complete T cell activation only in the presence of a particular molecular signature present at the tumor micro-environment;
FIG. 3 shows a Venn diagram of selection of three exemplary input signals **(a), (b)** and **(c)** that are specifically present at a tumor microenvironment.
FIG. 4 illustrates selection of three exemplary pancreatic cancer-specific input signals (**a** PSCA), (**b** TGFβ) and (**c** IL4) that are specifically present at a pancreatic tumor microenvironment.

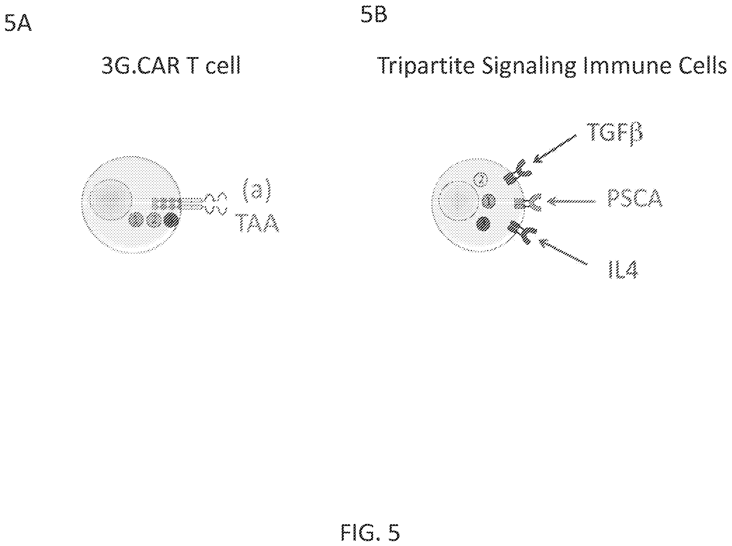

FIG. 5A shows a standard third generation CAR T cell and FIG. 5B shows an exemplary tripartite signaling cell of the disclosure. FIG. 5 demonstrates that while 3G.CAR T cells trigger three signals in response to a single input, the immune cells of the disclosure will require the combination of three independent signals in order to initiate the same signaling cascades.
FIG. 6 illustrates the non-exclusive distribution of the antigen expression. The identification of a tumor signature can help discriminate between normal and tumor tissue, given that tumor antigen expression in normal tissues can increase the risk of toxicity because of unwanted T cell expansion.
FIG. 7 shows the expansion of 3G.CAR T cells at the normal tissue site (PSCA only) vs. the expansion at the tumor site (PSCA, IL4, TGFβ).
FIG. 8 shows the preferential expansion of certain immune cells of the disclosure at the tumor site as opposed to the reduced expansion observed at the normal tissue site.
FIG. 9 shows the correlation between certain immune cells of the disclosure and tumor cell numbers in a co-culture experiment. Tumor growth was controlled by an increase in the number of the cells that reliably contracted upon tumor elimination.
FIG. 10 shows a co-culture of 3G.CAR T cells with target cells recreating normal tissue (Panel A) and tumor tissue (Panel B) during a period of 6 days. 3G.CAR T cells demonstrated a greater cytolytic activity and T cell expansion at the normal tissue (Panel A) *vs.* the tumor microenvironment (Panel B).
FIG. 11 shows a co-culture of certain immune cells of the disclosure with target cells recreating normal tissue (Panel A) and tumor tissue (Panel B) during a period of 6 days. The cells demonstrate a greater cytolytic activity and T cell expansion in conditions that mimic the tumor microenvironment (Panel B) while exhibiting a decreased cytolytic function and T cell expansion in the presence of the normal tissue (antigen only).
FIG. 12 shows expansion of particular immune cells in the presence of antigen (PSCA) and cytokine (IL4).
FIG. 13 shows expansion of bipartite signaling immune cells that are greater than 2G and 3G CARs when cultured with antigen and cytokine.
FIG. 14 shows the enrichment of the transgene for the bipartite signaling immune cells when cultured in the presence of antigen and IL4.
FIG. 15 shows the expansion of bipartite signaling immune cells in response to the combination of antigen and cytokine when compared to conditions with either antigen or cytokine alone.
FIG. 16 shows that an exemplary chimeric cytokine receptor 4/7R (IL4 receptor exodomain and an IL7 receptor endodomain) promotes proliferation of 1G CAR T cells in presence of IL4.
FIG. 17 demonstrates that 4/7R changes the gene expression profile of 1G CAR T cells after exposure to IL4.
FIG. 18 demonstrates that 4/7R retains a Th1 polarized cytokine expression profile in 1G T cells after exposure to IL4.
FIG. 19 demonstrates selective expansion of 4/7R transgenic T cells due to culture with IL-4.
FIG. 20 shows safety profile of 1G + 4/7R T cells as their expansion and persistence is antigen and cytokine dependent.
FIG. 21 demonstrates that T-BBR maintains cytolytic function of CAR T cells exposed to TGFβ.
FIG.22 shows that T-BBR modified CAR T cells expand and are thereby protected from TGFβ inhibition.
FIG. 23 illustrates that T-BBR alters gene expression of 1G CAR T cells after exposure to TGFβ.
FIG. 24 shows that T-BBR retains Bcl2 expression in T-BBR modified T cells after exposure to TGFβ.
FIG. 25 shows that 4-1BB endodomain maintains a Th1 polarized response in 1G + T-BBR T cells after exposure to TGFβ.
FIG. 26 demonstrates that 3G CAR T cells indiscriminately kill PSCA+ targets.
FIG. 27 illustrates that tripartite immune cells (comprising a signal for T cell activation, persistence, and cytokine release) exhibit preferential anti-tumor activity in presence of tumor signature (PSCA+ tumor cells in the presence of II,4 and TGFβ).
FIG. 28 shows comparison of the exemplary tripartite immune cells against 3G CAR T cells using a particular culturing system having a gas permeable bottom (G-Rex).
FIG. 29 demonstrates that the tripartite cells exhibit preferential anti-tumor activity against PSCA+, IL4+, TGFβ+ targets.
FIG. 30 shows preferential anti-tumor activity of the tripartite immune cells against PSCA, II,4 and TGFβ positive targets.

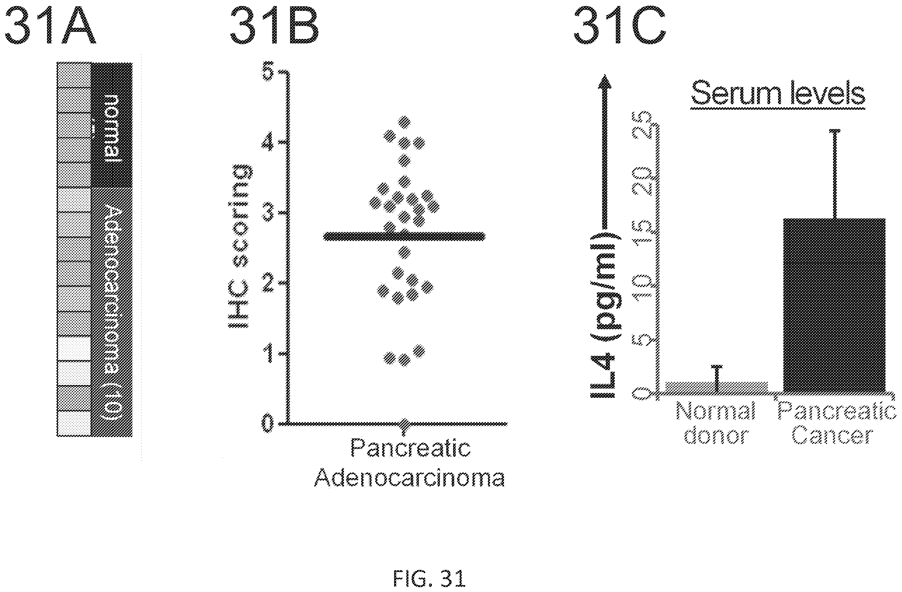

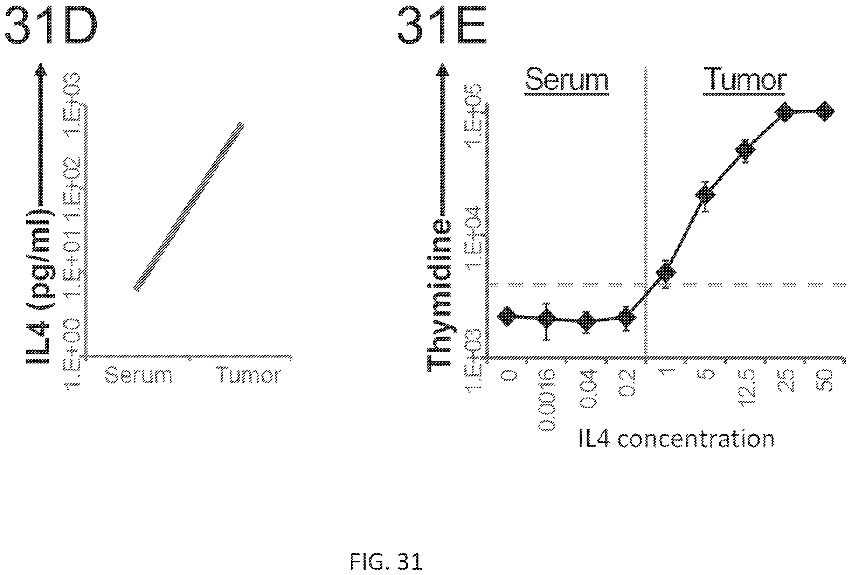

FIG. 31A provides illustration that Logsdon and colleagues performed gene expression profiling on pancreatic adenocarcinoma and normal pancreas demonstrating elevated IL4 levels in pancreatic tumors (fold change 19.78, p = 0.001) (Fig N1A). FIG. 31B demonstrates immunohistochemistry (IHC) studies scoring based on IL4 expression intensity confirmed such observations. FIG. 31C illustrates elevated IL4 cytokine levels in patient serum (n=7), as quantitatively measured by ELISA. FIG. 31D shows the IL4 cytokine concentration collected from the serum of mice engrafted with a IL4 producing tumor.. In FIG. 31E, 4/7R-modified cells were able to expand only in IL4 levels predicted to be present at the tumor.
FIG. 32 demonstrates that the presence of antigen and IL4 with bipartite T cells expressing 1G CAR and 4/7R results in upregulation of CD25.
FIG. 33 shows bipartite-modified T cells expressing 1G CAR and T-BBR were able to eliminate tumor cells in presence of TGFβ while in contrast, the administration of TGFβ inhibited the cytolytic function and prevented elimination of target cells by 1G CAR T cells alone.
FIG. 34 demonstrates transgenic expression of T-BBR protected CAR T cells from T cell exhaustion measured by down-regulation of PD-1 expression following administration of TGFβ.
FIG. 35 shows that double transgenic T cells expressing 1G CAR with T-BBR survive but do not expand in the presence of TGFβ.
FIG. 36 shows that bipartite T cells expressing T-BBR along with 2G CAR containing signals 1 and 2 resulted in T cell expansion in presence of TGFβ.
FIG. 37 demonstrates that tripartite T cells exhibit T cell expansion in the presence of the tumor molecular signature that can initiate signals 1, 2 and 3 simultaneously, whereas triggering signal 1, 2, or 3 independently results in insufficient T cell response.

### DETAILED DESCRIPTION

In keeping with long-standing patent law convention, the words "a" and "an" when used in the present specification in concert with the word comprising, including the claims, denote "one or more." Some aspects of the disclosure may consist of or consist essentially of one or more elements, method steps, and/or methods of the disclosure. It is contemplated that any method or composition described herein can be implemented with respect to any other method or composition described herein.

As used herein, the term "about" or "approximately" refers to a quantity, level, value, number, frequency, percentage, dimension, size, amount, weight or length that varies by as much as 30, 25, 20, 25, 10, 9, 8, 7, 6, 5, 4, 3, 2 or 1 % to a reference quantity, level, value, number, frequency, percentage, dimension, size, amount, weight or length. In particular aspects of the disclosure, the terms "about" or "approximately" when preceding a numerical value indicates the value plus or minus a range of 15%, 10%, 5%, or 1%.

Throughout this specification, unless the context requires otherwise, the words "comprise", "comprises" and "comprising" will be understood to imply the inclusion of a stated step or element or group of steps or elements but not the exclusion of any other step or element or group of steps or elements. By "consisting of" is meant including, and limited to, whatever follows the phrase "consisting of." Thus, the phrase "consisting of" indicates that the listed elements are required or mandatory, and that no other elements may be present. By "consisting essentially of" is meant including any elements listed after the phrase, and limited to other elements that do not interfere with or contribute to the activity or action specified in the disclosure for the listed elements. Thus, the phrase "consisting essentially of" indicates that the listed elements are required or mandatory, but that no other elements are optional and may or may not be present depending upon whether or not they affect the activity or action of the listed elements.

Reference throughout this specification to "one aspect," "an aspect," "a particular aspect," "a related aspect," "a certain aspect," "an additional aspect," "a further aspect," "a certain aspect," a particular aspect," "a specific aspect," or combinations thereof means that a particular feature, structure or characteristic described in connection with the aspect is included in at least one aspect of the present disclosure. Thus, the appearances of the foregoing phrases in various places throughout this specification are not necessarily all referring to the same aspect. Furthermore, the particular features, structures, or characteristics may be combined in any suitable manner in one or more aspects.

### I. Cells of the Disclosure

Encompassed in the disclosure are cells and methods related to the cells, wherein the cells comprise multiple signals of antigen stimulation, co-stimulation, and cytokine separated into different receptors, rather than incorporating all signals together into a single receptor. In certain embodiments, these cells are referred to as SmarT cells. In some aspects of the disclosure, bipartite signaling immune cells comprise two separate molecules to receive antigen stimulation and cytokine signaling, and in particular aspects co-stimulation occurs *via* and indirectly through the antigen stimulation input. In other aspects of the disclosure, there are tripartite signaling immune cells that utilize three separate molecules to receive antigen stimulation, co-stimulation, and cytokine signaling independent from each other. For example, the invention provides a composition, comprising an engineered T cell that separately expresses: 1) a chimeric antigen receptor (CAR) that provides antigen signaling for the cell upon binding of PSCA; 2) a co-stimulation receptor that provides co-stimulation for the cell, which is a chimeric cytokine receptor comprising a TGFβR exodomain and a 4-1BB endodomain; and 3) a cytokine receptor that provides cytokine stimulation for the cell, which is a chimeric cytokine receptor comprising an IL-4 receptor exodomain and an IL-7 receptor endodomain; wherein the antigen receptor and the co-stimulation receptor are different molecules.

In specific aspects of the disclosure, the immune cells are engineered cells. Although the immune cells naturally may have receptors for targeting antigens, receiving cytokine signals, and so forth, the immune cells of the present disclosure are non-natural and are engineered directly or indirectly by the hand of man such that they express the desired bipartite or tripartite signaling molecules. The engineered immune cells may be manipulated by recombinant engineering to express one, two, or three of the signaling molecules. Engineering of the cells to express one or more signaling molecules may occur in a single step or in multiple steps, and the manipulation may occur at a single point in time or in successive points in time.

In specific aspects, the cells are for adoptive transfer. The cells may be included in a pharmaceutical composition. The cells may be transformed or transfected with one or more vectors as described herein. The recombinant cells may be produced by introducing at least one of the vectors described herein. The presence of the vector in the cell mediates the expression of the appropriate receptor, and in some embodiments one or more constructs are integrated into the genome of the cell. That is, nucleic acid molecules or vectors that are introduced into the host may either integrate into the genome of the host or they may be maintained extrachromosomally.

As used herein, the terms "cell," "cell line," and "cell culture" may be used interchangeably, in particular embodiments. In certain aspects, these terms also include the cell's progeny, which includes any and all subsequent generations. It is understood that all progeny may not be identical due to deliberate or inadvertent mutations. In the context of expressing a heterologous nucleic acid sequence, "host cell" refers to a prokaryotic or eukaryotic cell, and it includes any transformable organism that is capable of replicating a vector and/or expressing a heterologous gene encoded by a vector. A host cell can, and has been, used as a recipient for vectors. A host cell may be "transfected" or "transformed," which refers to a process by which exogenous nucleic acid is transferred or introduced into the host cell. A transformed cell includes the primary subject cell and its progeny. In certain aspects, as used herein, the terms "engineered" and "recombinant" cells or host cells are intended to refer to a cell into which an exogenous nucleic acid sequence, such as, for example, a vector, has been introduced. Therefore, recombinant cells are distinguishable from naturally occurring cells that do not contain a recombinantly introduced nucleic acid.

In certain embodiments, it is contemplated that RNAs or proteinaceous sequences may be co-expressed with other selected RNAs or proteinaceous sequences in the same host cell. Co-expression may be achieved by co-transfecting the host cell with two or more distinct recombinant vectors. Alternatively, a single recombinant vector may be constructed to include multiple distinct coding regions for RNAs, which could then be expressed in host cells transfected with the single vector. In some cases, a cell may comprise one or more polynucleotides comprising one or more expression constructs.

Cells may comprise vectors that employ control sequences that allow them to be replicated and/or expressed in both prokaryotic and eukaryotic cells. One of skill in the art would further understand the conditions under which to incubate host cells to maintain them and to permit replication of a vector. Techniques and conditions that would allow large-scale production of cells contemplated herein are also known to those of skill in the art.

In some embodiments, expression of one or more of receptors of the engineered cells is regulated. The regulation of expression may include constitutive expression, inducible expression, environment-specific expression, or tissue-specific expression, and examples of such promoters are known in the art. The expression of different recombinant receptors in the cell may have the same or different types of promoters. Constitutive mammalian promoters include Simian virus 40, Immediate-early Cytomegalovirus virus, human ubiquitin C, elongation factor 1α-subunit, and Murine Phosphoglycerate Kinase-1, for example.

In particular aspects of the disclosure, the cells contemplated herein include eukaryotic cells, e.g., including mammalian cells. In certain aspects of the disclosure, the cells are human, but in particular embodiments the cells are equine, bovine, murine, ovine, canine, feline, *etc.* for use in their respective animal. Among these species, various types of cells can be involved, such as T-cells, NK-cells, NKT-cells, *etc.*

The cells can be autologous cells, syngeneic cells, allogeneic cells and even in some cases, xenogeneic cells with respect to the individual receiving them. The cells may be modified by changing the major histocompatibility complex ("MHC") profile, by inactivating β₂-microglobulin to prevent the formation of functional Class I MHC molecules, inactivation of Class II molecules, providing for expression of one or more MHC molecules, enhancing or inactivating cytotoxic capabilities by enhancing or inhibiting the expression of genes associated with the cytotoxic activity, or the like.

In some instances specific clones or oligoclonal cells may be of interest, where the cells have a particular specificity, such as T cells and B cells having a specific antigen specificity or homing target site specificity.

The exemplary T-cells may be modified in a way other than separately expressing multiple receptors. For example, one may wish to introduce genes encoding one or both chains of a T-cell receptor. For example, in addition to providing for expression of multiple genes having therapeutic value and, optionally, another therapeutic gene, in some embodiments the cell is modified to direct the cell to a particular site. The site can include anatomical sites, and in particular embodiments includes solid tumors. An increase in the localized concentrations of the cells can be achieved following their enhanced capability to migrate through the ECM by expressing surface membrane proteins on the host cell that will enable it to bind to a target site such as a naturally occurring epitope on a target cell. There are numerous situations where one would wish to direct cells to a particular site, where release of a therapeutic product could be of great value or where pathways in the cell are triggered that directly or indirectly result in apoptosis of the cell.

In one embodiment, the host cell is a T cell comprising separate expression of multiple receptors but also comprising an engineered αβTCR, a native receptor specific for a tumor antigen, and/or a CAR, for example. In certain cases one of the signaling receptors for the bipartite or tripartite immune cells described herein comprises an engineered αβTCR or a CAR.

Naturally occurring T cell receptors comprise two subunits, an α-subunit and a β-subunit, each of which is a unique protein produced by recombination event in each T cell's genome. Libraries of TCRs may be screened for their selectivity to particular target antigens. An "engineered TCR" may refer to a natural TCR, which has a high-avidity and reactivity toward target antigens that is selected, cloned, and/or subsequently introduced into a population of T cells used for adoptive immunotherapy. In contrast to engineered TCRs, CARs are engineered to bind target antigens in an MHC-independent manner. In particular embodiments, a CAR comprises an extracellular binding domain including, but not limited to, an antibody or antigen binding fragment thereof; a transmembrane domain; one or more intracellular costimulatory signaling domains and a primary signaling domain.

In various aspects described herein, an immune cell comprises separate expression of multiple receptors. In particular aspects described herein, a bipartite or tripartite signaling immune cell comprises one or more polynucleotides encoding the separate receptors of the bipartite or tripartite signals. Such cells may also express a CAR or engineered TCR, and the respective CAR or engineered TCR may be encoded by one or more polynucleotides. In specific aspects described herein, cytokine signaling for the immune cells concerns IL-15, IL-2, and/or IL-7. In other aspects described herein, the co-stimulatory domain is CD27, CD80, CD83, CD86, CD134, or CD137. In other specific embodiments, the exodomain is PD-1, PD-L1, CTLA4, or B7-H4.

Cells of the disclosure harboring an exogenous molecule(s) for expression of one of the bipartite or tripartite signal receptors may comprise a CAR as one of the signal receptors or separate from any of the signal receptors. The CAR generally comprises a tumor-associated antigen (TAA)-binding domain (most commonly a scFv derived from the antigen-binding region of a monoclonal antibody), an extracellular spacer/hinge region, a transmembrane domain and one or more intracellular signaling domains. The CAR may be first generation, second generation, or third generation (CAR in which signaling is provided by CD3ζ together with co-stimulation provided by one or more of CD28 and a tumor necrosis factor receptor (TNFr), such as 4-1BB or OX40), for example. In certain embodiments, the CAR lacks a costimulatory domain.

In some aspects of the disclosure, the CAR is specific for an antigen, for example one that is present in a tumor microenvironment. The CAR may be specific for EphA2, HER2, GD2, Glypican-3, 5T4, 8H9, αᵥβ₆ integrin, B cell maturation antigen (BCMA) B7-H3, B7-H6, CAIX, CA9, CD19, CD20, CD22, kappa light chain, CD30, CD33, CD38, CD44, CD44v6, CD44v7/8, CD70, CD123, CD138, CD171, CEA, CSPG4, EGFR, EGFRvIII, EGP2, EGP40, EPCAM, ERBB3, ERBB4, ErbB3/4, FAP, FAR, FBP, fetal AchR, Folate Receptor α, GD2, GD3, HLA-AI MAGE A1, HLA-A2, IL11Ra, IL13Ra2, KDR, Lambda, Lewis-Y, MCSP, Mesothelin, Muc1, Muc16, NCAM, NKG2D ligands, NY-ESO-1, PRAME, PSCA, PSC1, PSMA, ROR1, Sp17, SURVIVIN, TAG72, TEM1, TEM8, VEGRR2, carcinoembryonic antigen, HMW-MAA, VEGF receptors, and/or other exemplary antigens that are present with in the extracelluar matrix of tumors, such as oncofetal variants of fibronectin, tenascin, or necrotic regions of tumors. The CAR (by way of example only) and one or more of the bipartite or tripartite signal receptors may be on the same or different vectors. In one aspects of the disclosure, a cell comprises one or more vectors encoding a CAR and one or more cytokines (such as IL-2, IL-7, or IL-15, for example). Chimeric antigen structure and nomenclature is known in the art, *e.g.,* see U.S. Patent Nos. 7,741,465; 5,906,936; 5,843,728; 6,319,494; 7,446,190; 5,686,281; 8,399,645; and U.S. Patent Application Publication Nos. 2012/0148552.

In particular aspects of the disclosure, the immune cell comprises one or more chimeric cytokine receptors that comprise an exodomain and an endodomain that are not naturally of the same molecule. In specific aspects of the disclosure, the exodomain is from the exodomain of a receptor. The exodomain of the chimeric cytokine receptor binds a cytokine, in particular embodiments. The exodomain may be from a receptor or molecule that can bind to TGFβ, IL10, IL4, IL13, IL6, IL8, IL5, VEGF, IL22, IL1, IL1β, IL35, TNF, GM-CSF, M-CSF, G-CSF, for example. The exodomain may be from a chemokine receptor. In specific embodiments, the exodomain is from TGFβRII, although it may instead be from TGFβRI or TGFβRIII. For the endodomain component of the chimeric cytokine receptor, the endodomain may be a signal transducing endodomain. Examples of signal transducing domains include endodomains from TLR1,TLR2, TLR3, TLR4, TLR5, TLR6, TLR7, TLR8, TLR9, CD28, OX-40, 4-1BB, CD80, CD86, ICOS, CD40, CD27, CD30, CD226, IL7, IL2, IL15, IL21, IL12, IL18, IL9 and IFN-gamma.

Although in many cases the immune cells of the disclosure will stop proliferating once one or more of the signals is reduced or eliminated from a tumor microenvironment (such as upon killing of the cancer cells), in some aspects of the disclosure it may be desirable to kill the engineered immune cells, such as when the object is to terminate the treatment, the cells become neoplastic, in a research setting, and/or another event. In particular aspects of the disclosure, the engineered cell comprises a suicide gene, optionally regulated at the level of transcription, translation, or post-translationally. Suicide genes are known in the art, e.g., the iCaspase9 system in which a modified form of caspase 9 is dimerizable with a small molecule, *e.g.,* AP1903. See, *e.g.,* Straathof et al., Blood 105:4247-4254 (2005).

### II. Therapeutic Uses of the Cells

In one embodiment, engineered immune cells are used for the prevention, treatment or amelioration of a cancerous disease, such as a tumorous disease. In particular embodiments, the pharmaceutical composition contemplated herein may be particularly useful in preventing, ameliorating and/or treating cancers (or ameliorating one or more symptoms thereof) in which having the multiple separately expressed receptors renders the cells of the pharmaceutical composition more effective than if the cells lacked expression of the receptor(s). In specific embodiments, cancer cells being treated with pharmaceutical compositions are effectively treated because cells of the pharmaceutical compositions have selective expression in a tumor microenvironment. In particular embodiments, the cancer is in the form of a solid tumor.

As used herein "treatment" or "treating," includes any beneficial or desirable effect on the symptoms or pathology of a disease or pathological condition, and may include even minimal reductions in one or more measurable markers of the disease or condition being treated, e.g., cancer. Treatment can involve optionally either the reduction or amelioration of symptoms of the disease or condition, or the delaying of the progression of the disease or condition. "Treatment" does not necessarily indicate complete eradication or cure of the disease or condition, or associated symptoms thereof.

As used herein, "prevent," and similar words such as "prevented," "preventing" *etc.,* indicate an approach for preventing, inhibiting, or reducing the likelihood of the occurrence or recurrence of, a disease or condition, *e.g*., cancer. It also refers to delaying the onset or recurrence of a disease or condition or delaying the occurrence or recurrence of the symptoms of a disease or condition. As used herein, "prevention" and similar words also includes reducing the intensity, effect, symptoms and/or burden of a disease or condition prior to onset or recurrence of the disease or condition.

An individual may be subjected to compositions of the disclosure that is at risk for a solid tumor. The individual may be at risk because of having one or more known risk factors, such as family or personal history, being a smoker, having one or more genetic markers, and so forth.

Possible indications for administration of the composition(s) of the immune cells are cancerous diseases, including tumorous diseases, including cancer of the breast, brain, bone, prostate, lung, colon, head and neck, skin, ovary, endometrium, cervix, kidney, lung, stomach, small intestine, liver, pancreas, testis, pituitary gland, blood, spleen, gall bladder, bile duct, esophagus, salivary glands and the thyroid gland, for example. In particular embodiments, the administration of the composition(s) of the disclosure is useful for all stages and types of cancer, including for minimal residual disease, early cancer, advanced cancer, and/or metastatic cancer and/or refractory cancer, for example.

The disclosure further encompasses co-administration protocols with other compounds that are effective against cancer. The clinical regimen for co-administration of the inventive cell(s) may encompass co-administration at the same time, before, or after the administration of the other component. Particular combination therapies include chemotherapy, radiation, surgery, hormone therapy, or immunotherapy, or combinations thereof, for example.

By way of illustration, cancer patients or patients susceptible to cancer or suspected of having cancer may be treated as follows. Engineered cells may be administered to the patient and retained for extended periods of time. The individual may receive one or more administrations of the cells. Illustrative cells include *ex vivo* expanded T-cells. In various embodiments, the cell is modified at least to express the tripartite signaling receptors and is provided to the individual in need thereof in effective amount. In some embodiments, the cells may be injected directly into the tumor.

In some embodiments, the genetically modified cells are encapsulated to inhibit immune recognition and are placed at the site of the tumor. For example, the cells may be encapsulated in liposomes, alginate, or platelet-rich plasma.

In another embodiment, antigen-specific T cells may be modified to export hormones or factors that are exocytosed. By providing for enhanced exocytosis, a greater amount of the hormone or factor will be exported; in addition, if there is a feedback mechanism based on the amount of the hormone or factor in the cytoplasm, increased production of the hormone or factor will result. In one aspect, one may provide for induced expression of the hormone or factor, so that expression and export may be induced concomitantly.

### III. Introduction of Constructs into Cells

The different expression constructs can be introduced as one or more polynucleotides or constructs, optionally comprising a marker that will allow for selection of host cells that contain the construct(s), and in specific embodiments each receptor is expressed from a different expression construct. In some embodiments, a polynucleotide encodes the separate signaling polypeptides from the same construct, and in some embodiments, a polynucleotide encodes the separate signaling polypeptides on different constructs.

The constructs can be prepared in conventional ways, where the genes and regulatory regions may be isolated, as appropriate, ligated, cloned in an appropriate cloning host, analyzed by restriction or sequencing, or other convenient means. Particularly, using PCR, individual fragments including all or portions of a functional unit may be isolated, where one or more mutations may be introduced using "primer repair", ligation, *in vitro* mutagensis, *etc.* as appropriate. The construct(s) once completed and demonstrated to have the appropriate sequences may then be introduced into the host cell by any convenient means. The constructs may be integrated and packaged into non-replicating, defective viral genomes like lentivirus, Adenovirus, Adeno-associated virus (AAV), or Herpes simplex virus (HSV) or others, including retroviral vectors, for infection or transduction into cells. The constructs may include viral sequences for transfection, if desired. Alternatively, the construct may be introduced by fusion, electroporation, biolistics, transfection, lipofection, or the like. The host cells may be grown and expanded in culture before introduction of the construct(s), followed by the appropriate treatment for introduction of the construct(s) and integration of the construct(s). The cells are then expanded and screened by virtue of a marker present in the construct. Various markers that may be used successfully include hprt, neomycin resistance, thymidine kinase, hygromycin resistance, *etc.*

In specific embodiments, the receptors are introduced into the cells as an RNA for transient expression. RNA can be delivered to the immune cells of the disclosure by various means including microinjection, electroporation, and lipid-mediated transfection, for example. In particular aspects, introduction of constructs into the cell's genome may occur *via* transposons. An example of a synthetic transposon for use is the Sleeping Beauty transposon that comprises an expression cassette including the appropriate gene of active fragment thereof.

In some instances, one may have a target site for homologous recombination, where it is desired that a construct be integrated at a particular locus. For example,) an endogenous gene can be replaced with the gene encoded for by the construct using materials and methods as are known in the art for homologous recombination. In particular embodiments, one may use either .OMEGA, or O-vectors to achieve homologous recombination. See, for example, Thomas and Capecchi, 1987; Mansour, *et al.,* 1988; and Joyner, *et al.,* 1989.

The constructs may be introduced as a single DNA molecule encoding at least one receptor and optionally another gene, or different DNA molecules having one or more genes. The constructs may be introduced simultaneously or consecutively, each with the same or different markers. In an illustrative example, one construct would encode a signaling molecule and control particular expression control sequences.

Vectors containing useful elements such as bacterial or yeast origins of replication, selectable and/or amplifiable markers, promoter/enhancer elements for expression in prokaryotes or eukaryotes, *etc.* that may be used to prepare stocks of construct DNAs and for carrying out transfections are well known in the art, and many are commercially available.

### IV. Administration of Cells

The cells that have been modified to express the receptors (such as with DNA constructs) may be grown in culture under selective conditions, and cells that are selected as having the constructs may then be expanded and further analyzed, using, for example; the polymerase chain reaction for determining the presence of the construct in the host cells. Once the modified host cells have been identified, they may then be used as planned, *e.g*. expanded in culture or introduced into a host organism.

Depending upon the nature of the cells, the cells may be introduced into a host organism, *e.g.* a mammal, in a wide variety of ways. The cells are introduced at the site of the tumor, in specific embodiments, although in alternative embodiments the cells hone to the cancer or are modified to hone to the cancer, such as upon systemic administration, for example. The number of cells that are employed will depend upon a number of circumstances, the purpose for the introduction, the lifetime of the cells, the protocol to be used, for example, the number of administrations, the ability of the cells to multiply, the stability of the recombinant construct, and the like. The cells may be applied as a dispersion, generally being injected at or near the site of interest. The cells may be in a physiologically-acceptable medium.

The DNA introduction need not result in integration into the host cell genome in every case. In some situations, transient episomal maintenance of the DNA may be sufficient. In this way, one could have a short-term effect, where cells could be introduced into the host and then turned on after a predetermined time, for example, after the cells have been able to home to a particular site.

The cells may be administered to an individual in need thereof. Administration of the cells may depend upon the response desired, the manner of administration, the life of the cells, the number of cells present, various protocols may be employed. The number of administrations will depend upon the factors described herein at least in part. In particular embodiments, the route of administration may be intravenous, intraarterial, intraperitoneal, or subcutaneous, for example. Multiple administrations may be by the same route or by different routes. Determination of appropriate dose levels are routinely performed in the art.

In particular embodiments, a plurality of immune cells are delivered to an individual with cancer. In specific embodiments, a single administration is performed. In other embodiments, a plurality of administration of cells is performed. For example, following a first, second, third, or more administration of the engineered immune cells, there may be examination of the individual for the presence or absence of the cancer or for a reduction in the number and/or size of tumors, for example. In the event that the cancer shows a need for further treatment, such as upon tumor growth after the first or subsequent administration, an additional one or more deliveries of the same engineered immune cells (or, optionally, another type of cancer therapy, including another type of immunotherapy, and/or chemotherapy, surgery and/or radiation) is given to the individual. When two or more administrations of cells are provided to the individual, the duration in time between the administrations may be of any suitable time, including on the order of days (such as 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, or more), weeks (such as 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, or more), months (such as 1, 2, 3, 4, 5, 6, 7, 8, 9, 10, 11, 12, or more), or years (such as 1, 2, 3, 4, 5, 6, 7, 8, 9, 10 or more). In some cases, a reduction of tumor size in an individual indicates that the particular immunotherapy is effective, so further administrations of the same cells are provided to the individual.

It should be appreciated that the system is subject to variables, such as the cellular response to the ligand, the efficiency of expression and, as appropriate, the level of secretion, the activity of the expression product, the particular need of the patient, which may vary with time and circumstances, the rate of loss of the cellular activity as a result of loss of cells or expression activity of individual cells, and the like. Therefore, it is expected that for each individual patient, even if there were universal cells that could be administered to the population at large, each patient would be monitored for the proper dosage for the individual, and such practices of monitoring a patient are routine in the art.

### V. Polynucleotides Encoding Signaling Molecules for Bipartite or Tripartite Immune Cells

The present disclosure also encompasses a composition comprising a nucleic acid sequence encoding signaling molecules for bipartite or tripartite signaling immune cells as defined herein and cells harboring the nucleic acid sequence. The nucleic acid molecule is a recombinant nucleic acid molecule, in particular embodiments. In particular embodiments, the nucleic acid molecule is synthetic. It may comprise DNA, RNA as well as PNA (peptide nucleic acid) and it may be a hybrid thereof.

It is evident to the person skilled in the art that one or more regulatory sequences may be added to the nucleic acid molecule described herein. For example, promoters, transcriptional enhancers and/or sequences that allow for induced expression of the polynucleotide of the disclosure may be employed. A suitable inducible system is for example tetracycline-regulated gene expression as described, *e.g*., by Gossen and Bujard (Proc. Natl. Acad. Sci. USA 89 (1992), 5547-5551) and Gossen et al. (Trends Biotech. 12 (1994), 58-62), or a dexamethasone-inducible gene expression system as described, *e.g.* by Crook (1989) EMBO J. 8, 513-519.

Furthermore, it is envisaged for further purposes that nucleic acid molecules may contain, for example, thioester bonds and/or nucleotide analogues. The modifications may be useful for the stabilization of the nucleic acid molecule against endo- and/or exonucleases in the cell. The nucleic acid molecules may be transcribed by an appropriate vector comprising a chimeric gene that allows for the transcription of said nucleic acid molecule in the cell. In this respect, it is also to be understood that such polynucleotides can be used for "gene targeting" or "gene therapeutic" approaches. In another aspect of the disclosure, the nucleic acid molecules are labeled. Methods for the detection of nucleic acids are well known in the art, e.g., Southern and Northern blotting, PCR or primer extension. This may be useful for screening methods for verifying successful introduction of the nucleic acid molecules described above during gene therapy approaches.

The nucleic acid molecule(s) may be a recombinantly produced chimeric nucleic acid molecule comprising any of the aforementioned nucleic acid molecules either alone or in combination. In specific aspects of the disclosure, the nucleic acid molecule is part of a vector.

The present disclosure therefore also relates to a composition comprising a vector comprising the nucleic acid molecule described in the present disclosure.

Many suitable vectors are known to those skilled in molecular biology, the choice of which would depend on the function desired and include plasmids, cosmids, viruses, bacteriophages and other vectors used conventionally in genetic engineering. Methods that are well known to those skilled in the art can be used to construct various plasmids and vectors; see, for example, the techniques described in Sambrook *et al.* (1989) and Ausubel, Current Protocols in Molecular Biology, Green Publishing Associates and Wiley Interscience, N.Y. (1989), (1994). Alternatively, the polynucleotides and vectors of the disclosure can be reconstituted into liposomes for delivery to target cells. A cloning vector may be used to isolate individual sequences of DNA. Relevant sequences can be transferred into expression vectors where expression of a particular polypeptide is required. Typical cloning vectors include pBluescript SK, pGEM, pUC9, pBR322 and pGBT9. Typical expression vectors include pTRE, pCAL-n-EK, pESP-1, pOP13CAT.

In specific aspects of the disclosure, there is a vector that comprises a nucleic acid sequence that is a regulatory sequence operably linked to the nucleic acid sequence encoding a chimeric cytokine receptor construct defined herein. Such regulatory sequences (control elements) are known to the artisan and may include a promoter, a splice cassette, translation initiation codon, translation and insertion site for introducing an insert into the vector. In specific aspects of the disclosure, the nucleic acid molecule is operatively linked to said expression control sequences allowing expression in eukaryotic or prokaryotic cells.

It is envisaged that a vector is an expression vector comprising the nucleic acid molecule encoding a polypeptide contemplated herein. In specific aspects, the vector is a viral vector, such as a lentiviral vector. Lentiviral vectors are commercially available, including from Clontech (Mountain View, CA) or GeneCopoeia (Rockville, MD), for example.

The terms "regulatory sequence" or "expression control sequence" refers to DNA sequences that are necessary to effect the expression of coding sequences to which they are ligated. The nature of such control sequences differs depending upon the host organism. In prokaryotes, control sequences generally include promoters, ribosomal binding sites, and terminators. In eukaryotes generally control sequences include promoters, terminators and, in some instances, enhancers, transactivators or transcription factors. The term "expression control sequence" is intended to include, at a minimum, all components the presence of which are necessary for expression, and may also include additional advantageous components.

The term "operably linked" refers to a juxtaposition wherein the components so described are in a relationship permitting them to function in their intended manner. A control sequence "operably linked" to a coding sequence is ligated in such a way that expression of the coding sequence is achieved under conditions compatible with the control sequences. In case the control sequence is a promoter, a double-stranded nucleic acid is preferably used.

Thus, the recited vector is an expression vector, in certain aspects described herein. An "expression vector" is a construct that can be used to transform a selected host and provides for expression of a coding sequence in the selected host. Expression vectors can for instance be cloning vectors, binary vectors or integrating vectors. Expression comprises transcription of the nucleic acid molecule preferably into a translatable mRNA. Regulatory elements ensuring expression in prokaryotes and/or eukaryotic cells are well known to those skilled in the art. In the case of eukaryotic cells they comprise normally promoters ensuring initiation of transcription and optionally poly-A signals ensuring termination of transcription and stabilization of the transcript. Possible regulatory elements permitting expression in prokaryotic host cells comprise, *e.g.*, the P_{L}, lac, trp or tac promoter in *E. coli,* and examples of regulatory elements permitting expression in eukaryotic host cells are the AOX1 or GAL1 promoter in yeast or the CMV-, SV40-, RSV-promoter (Rous sarcoma virus), CMV-enhancer, SV40-enhancer or a globin intron in mammalian and other animal cells.

Beside elements that are responsible for the initiation of transcription such regulatory elements may also comprise transcription termination signals, such as the SV40-poly-A site or the tk-poly-A site, downstream of the polynucleotide. Furthermore, depending on the expression system used leader sequences capable of directing the polypeptide to a cellular compartment or secreting it into the medium may be added to the coding sequence of the recited nucleic acid sequence and are well known in the art. The leader sequence(s) is (are) assembled in appropriate phase with translation, initiation and termination sequences, and preferably, a leader sequence capable of directing secretion of translated protein, or a portion thereof, into the periplasmic space or extracellular medium. Optionally, the heterologous sequence can encode a fusion protein including an N-terminal identification peptide imparting desired characteristics, *e.g.*, stabilization or simplified purification of expressed recombinant product; see supra. In this context, suitable expression vectors are known in the art such as Okayama-Berg cDNA expression vector pcDV1 (Pharmacia), pEF-Neo, pCDM8, pRc/CMV, pcDNA1, pcDNA3 (Invitrogen), pEF-DHFR and pEF-ADA, (Raum et al. Cancer Immunol Immunother (2001) 50(3), 141-150) or pSPORT1 (GIBCO BRL).

In some aspects of the disclosure, the expression control sequences are eukaryotic promoter systems in vectors capable of transforming of transfecting eukaryotic host cells, but control sequences for prokaryotic hosts may also be used. Once the vector has been incorporated into the appropriate host, the host is maintained under conditions suitable for high level expression of the nucleotide sequences, and as desired, the collection and purification of the polypeptide of the disclosure may follow.

Additional regulatory elements may include transcriptional as well as translational enhancers. In particular embodiments, vectors comprises a selectable and/or scorable marker. Selectable marker genes useful for the selection of transformed cells are well known to those skilled in the art and comprise, for example, antimetabolite resistance as the basis of selection for dhfr, which confers resistance to methotrexate (Reiss, Plant Physiol. (Life-Sci. Adv.) 13 (1994), 143-149); npt, which confers resistance to the aminoglycosides neomycin, kanamycin and paromycin (Herrera-Estrella, EMBO J. 2 (1983), 987-995) and hygro, which confers resistance to hygromycin (Marsh, Gene 32 (1984), 481-485). Additional selectable genes have been described, namely trpB, which allows cells to utilize indole in place of tryptophan; hisD, which allows cells to utilize histinol in place of histidine (Hartman, Proc. Natl. Acad. Sci. USA 85 (1988), 8047); mannose-6-phosphate isomerase which allows cells to utilize mannose (WO 94/20627) and ODC (omithine decarboxylase) which confers resistance to the ornithine decarboxylase inhibitor, 2-(difluoromethyl)-DL-omithine, DFMO (McConlogue, 1987, In: Current Communications in Molecular Biology, Cold Spring Harbor Laboratory ed.) or deaminase from Aspergillus terreus which confers resistance to Blasticidin S (Tamura, Biosci. Biotechnol. Biochem. 59 (1995), 2336-2338).

Useful scorable markers are also known to those skilled in the art and are commercially available. Advantageously, said marker is a gene encoding luciferase (Giacomin, Pl. Sci. 116 (1996), 59-72; Scikantha, J. Bact. 178 (1996), 121), green fluorescent protein (Gerdes, FEBS Lett. 389 (1996), 44-47) or β-glucuronidase (Jefferson, EMBO J. 6 (1987), 3901-3907). This is particularly useful for simple and rapid screening of cells, tissues and organisms containing a recited vector.

As described above, the recited nucleic acid molecule can be used in a cell, alone, or as part of a vector to express the encoded polypeptide in cells. The nucleic acids or vectors containing the DNA sequence(s) encoding any one of the above described chimeric cytokine receptor constructs is introduced into the cells that in turn produce the polypeptide of interest. The recited nucleic acids and vectors may be designed for direct introduction or for introduction *via* liposomes, or viral vectors (*e.g*., adenoviral, retroviral) into a cell. In certain embodiments, the cells are T-cells, CAR T-cells, NK cells, NKT-cells, MSCs, neuronal stem cells, or hematopoietic stem cells, for example.

In accordance with the above, the present disclosure relates to methods to derive vectors, particularly plasmids, cosmids, viruses and bacteriophages used conventionally in genetic engineering that comprise a nucleic acid molecule encoding the polypeptide sequence of a chimeric cytokine receptor construct contemplated herein. In particular aspects of the disclosure, the vector is an expression vector and/or a gene transfer or targeting vector. Expression vectors derived from viruses such as retroviruses, vaccinia virus, adeno-associated virus, herpes viruses, or bovine papilloma virus, may be used for delivery of the recited polynucleotides or vector into targeted cell populations. Methods which are well known to those skilled in the art can be used to construct recombinant vectors; see, for example, the techniques described in Sambrook *et al.* (loc cit.), Ausubel (1989, loc cit.) or other standard text books. Alternatively, the recited nucleic acid molecules and vectors can be reconstituted into liposomes for delivery to target cells. The vectors containing the nucleic acid molecules of the disclosure can be transferred into the host cell by well-known methods, which vary depending on the type of cellular host. For example, calcium chloride transfection is commonly utilized for prokaryotic cells, whereas calcium phosphate treatment or electroporation may be used for other cellular hosts; see Sambrook, supra.

### VI. Pharmaceutical Compositions

The term "pharmaceutical composition" relates to a composition for administration to an individual and encompasses compositions of cells for immunotherapy. In one embodiment, the invention provides a composition of the invention, for use in a method of treating an individual in need of immunotherapy for a medical condition, comprising the step of delivering a therapeutically effective amount of the composition to the individual.

In other aspects of the disclosure, the cells for immunotherapy are engineered to express at least one signaling molecule. In certain aspects of the disclosure, the cells comprise one or more modifications in addition to the at least one signaling molecule, such as one or more receptors, including artificial and natural receptors, for example receptors for tumor antigens. Particular receptors include a CAR or an engineered αβTCR, although in some cases the cell comprises a native TCR. In certain aspects of the disclosure, the composition comprises a CAR. In particular embodiments, the CAR comprises a costimulatory domain. In other aspects of the disclosure, the CAR does not comprise or lacks a costimulatory domain.

In further aspects of the disclosure, the cells for immunotherapy are engineered to express more than one separate CAR molecules, such as three or at least three separate CAR molecules. In certain aspects of the disclosure, the cells comprise one or more modifications in addition to the at least one signaling molecule, such as one or more receptors, including artificial and natural receptors, for example receptors for tumor antigens. Particular receptors include a CAR or an engineered αβTCR, although in some cases the cell comprises a native TCR. In certain aspects of the disclosure, the composition comprises a CAR. In particular aspects of the disclosure, the CAR comprises a costimulatory domain. In other embodiments, the CAR does not comprise or lacks a costimulatory domain.

In a particular embodiment, the pharmaceutical composition comprises a composition for parenteral, transdermal, intraluminal, intra-arterial, intrathecal or intravenous administration or for direct injection into a cancer. In one embodiment, the pharmaceutical composition is administered to the individual *via* infusion or injection. Administration of the suitable compositions may be effected by different ways, *e.g*., by intravenous, subcutaneous, intraperitoneal, intramuscular, topical or intradermal administration.

The pharmaceutical composition may further comprise a pharmaceutically acceptable carrier. Examples of suitable pharmaceutical carriers are well known in the art and include phosphate buffered saline solutions, water, emulsions, such as oil/water emulsions, various types of wetting agents, sterile solutions, *etc.* Compositions comprising such carriers can be formulated by well-known conventional methods. These pharmaceutical compositions can be administered to the subject at a suitable dose.

The dosage regimen will be determined by the attending physician and clinical factors. As is well known in the medical arts, dosages for any one patient depends upon many factors, including the patient's size, body surface area, age, the particular compound to be administered, sex, time and route of administration, general health, and other drugs being administered concurrently.

The compositions of the disclosure may be administered locally or systemically. Administration will generally be parenteral, *e.g.*, intravenous; DNA may also be administered directly to the target site, *e.g.*, by biolistic delivery to an internal or external target site or by catheter to a site in an artery. In a preferred embodiment, the pharmaceutical composition is administered subcutaneously and in an even more preferred embodiment intravenously. Preparations for parenteral administration include sterile aqueous or non-aqueous solutions, suspensions, and emulsions. Examples of non-aqueous solvents are propylene glycol, polyethylene glycol, vegetable oils such as olive oil, and injectable organic esters such as ethyl oleate. Aqueous carriers include water, alcoholic/aqueous solutions, emulsions or suspensions, including saline and buffered media. Parenteral vehicles include sodium chloride solution, Ringer's dextrose, dextrose and sodium chloride, lactated Ringer's, or fixed oils. Intravenous vehicles include fluid and nutrient replenishes, electrolyte replenishers (such as those based on Ringer's dextrose), and the like. Preservatives and other additives may also be present such as, for example, antimicrobials, anti-oxidants, chelating agents, and inert gases and the like. In addition, the pharmaceutical composition of the present disclosure might comprise proteinaceous carriers, like, *e.g.*, serum albumin or immunoglobulin, preferably of human origin. In certain embodiments, the pharmaceutical composition of the disclosure comprises, in addition to the proteinaceous chimeric cytokine receptor constructs or nucleic acid molecules or vectors encoding the same (as described in this disclosure), further biologically active agents, depending on the intended use of the pharmaceutical composition.

### VII. Kits

Any of the compositions described herein may be comprised in a kit. In a non-limiting example, one or more bipartite or tripartite signaling immune cells for use in cell therapy may be comprised in a kit. The kit components are provided in suitable container means.

For example, the invention provides a kit comprising the composition of the invention, said composition housed in a suitable container.

In specific embodiments, the kits comprise recombinant engineering reagents, such as vectors, primers, enzymes (restriction enzymes, ligase, polymerases, *etc.*), buffers, nucleotides, *etc.*

Some components of the kits may be packaged either in aqueous media or in lyophilized form. The container means of the kits will generally include at least one vial, test tube, flask, bottle, syringe or other container means, into which a component may be placed, and preferably, suitably aliquoted. Where there are more than one component in the kit, the kit also will generally contain a second, third or other additional container into which the additional components may be separately placed. However, various combinations of components may be comprised in a vial. The kits of the present disclosure also will typically include a means for containing the components in close confinement for commercial sale. Such containers may include injection or blow-molded plastic containers into which the desired vials are retained.

When the components of the kit are provided in one and/or more liquid solutions, the liquid solution is an aqueous solution, with a sterile aqueous solution being particularly useful. In some cases, the container means may itself be a syringe, pipette, and/or other such like apparatus, from which the formulation may be applied to an infected area of the body, injected into an animal, and/or even applied to and/or mixed with the other components of the kit.

However, the components of the kit may be provided as dried powder(s). When reagents and/or components are provided as a dry powder, the powder can be reconstituted by the addition of a suitable solvent. It is envisioned that the solvent may also be provided in another container means. The kits may also comprise a second container means for containing a sterile, pharmaceutically acceptable buffer and/or other diluent.

In particular embodiments, cells that are to be used for cell therapy are provided in a kit, and in some cases the cells are essentially the sole component of the kit. The kit may comprise in addition reagents and materials to make the cell recombinant for one or more signaling molecules. In specific embodiments, the reagents and materials include primers for amplifying a signaling molecule, nucleotides, suitable buffers or buffer reagents, salt, and so forth, and in some cases the reagents include vectors and/or DNA that encodes or more signaling molecules and/or regulatory elements therefor. Examples of reagents also include buffers and other materials for cell culture. In specific embodiments, one or more cytokines are provided in the kit.

In particular embodiments, there are one or more apparatuses in the kit suitable for extracting one or more samples from an individual. The apparatus may be a syringe, scalpel, and so forth.

In some embodiments, the kit includes one or more reagents or apparatus for diagnosis of cancer, including histological reagents, antibodies, blood or urine analysis reagents, and so forth.

In other embodiments, the kit, in addition to cell therapy embodiments, also includes a second cancer therapy, such as chemotherapy, hormone therapy, and/or immunotherapy, for example. The kit(s) may be tailored to a particular cancer for an individual and comprise respective second cancer therapies for the individual.

In some embodiments, the cell in the kit may be modified to express a therapeutic molecule other than a signaling molecule. The other therapeutic molecule may be of any kind, but in specific embodiments, the therapeutic molecule is a chimeric antigen receptor, for example.

In some embodiments, the kit, in addition to cell therapy embodiments, also includes a second cancer therapy, such as chemotherapy, hormone therapy, and/or immunotherapy, for example. The kit(s) may be tailored to a particular cancer for an individual and comprise respective second cancer therapies for the individual.

### VIII. Combination Therapy

In certain embodiments of the disclosure, compositions of the present disclosure for clinical aspects are combined with other agents effective in the treatment of hyperproliferative disease, such as anti-cancer agents (which may also be referred to as a cancer therapy).

An "anti-cancer" agent is capable of negatively affecting cancer in a subject, for example, by killing cancer cells, inducing apoptosis in cancer cells, reducing the growth rate of cancer cells, reducing the incidence or number of metastases, reducing tumor size, inhibiting tumor growth, reducing the blood supply to a tumor or cancer cells, promoting an immune response against cancer cells or a tumor, preventing or inhibiting the progression of cancer, or increasing the lifespan of a subject with cancer. More generally, these other compositions would be provided in a combined amount effective to kill or inhibit proliferation of the cell. This process may involve contacting the cancer cells with the expression construct and the agent(s) or multiple factor(s) at the same time. This may be achieved by contacting the cell with a single composition or pharmacological formulation that includes both agents, or by contacting the cell with two distinct compositions or formulations, at the same time, wherein one composition includes the expression construct and the other includes the second agent(s).

Tumor cell resistance to chemotherapy and radiotherapy agents represents a major problem in clinical oncology. One goal of current cancer research is to find ways to improve the efficacy of chemo- and radiotherapy by combining it with gene therapy. For example, the herpes simplex-thymidine kinase (HS-tK) gene, when delivered to brain tumors by a retroviral vector system, successfully induced susceptibility to the antiviral agent ganciclovir (Culver, *et al.,* 1992). In particular embodiments, it is contemplated that cell therapy could be used similarly in conjunction with chemotherapeutic, radiotherapeutic, or immunotherapeutic intervention, in addition to other pro-apoptotic or cell cycle regulating agents.

In another embodiment, therapy may precede or follow the other agent treatment by intervals ranging from minutes to weeks. In embodiments where the other agent and cellular therapy are applied separately to the individual, one would generally ensure that a significant period of time did not expire between the time of each delivery, such that the agent and cellular therapy would still be able to exert an advantageously combined effect on the tumor cell. In such instances, it is contemplated that one may contact the tumor cell with both modalities within about 12-24 h of each other and, more preferably, within about 6-12 h of each other. In some situations, it may be desirable to extend the time period for treatment significantly, however, where several d (2, 3, 4, 5, 6 or 7) to several wk (1, 2, 3, 4, 5, 6, 7 or 8) lapse between the respective administrations.

Various combinations may be employed, present disclosure is "A" and the secondary agent, such as radio- or chemotherapy, is "B":
A/B/A B/A/B B/B/A A/A/B A/B/B B/A/A A/B/B/B B/A/B/B
B/B/B/A B/B/A/B A/A/B/B A/B/A/B A/B/B/A B/B/A/A
B/A/B/A B/A/A/B A/A/A/B B/A/A/A A/B/A/A A/A/B/A

It is expected that the treatment cycles would be repeated as necessary. It also is contemplated that various standard therapies, as well as surgical intervention, may be applied in combination with the inventive cell therapy.

### A. Chemotherapy

Cancer therapies also include a variety of combination therapies with both chemical and radiation based treatments. Combination anti-cancer agents include, for example, acivicin; aclarubicin; acodazole hydrochloride; acronine; adozelesin; aldesleukin; altretamine; ambomycin; ametantrone acetate; amsacrine; anastrozole; anthramycin; asparaginase; asperlin; azacitidine; azetepa; azotomycin; batimastat; benzodepa; bicalutamide; bisantrene hydrochloride; bisnafide dimesylate; bizelesin; bleomycin sulfate; brequinar sodium; bropirimine; busulfan; cactinomycin; calusterone; caracemide; carbetimer; carboplatin; carmustine; carubicin hydrochloride; carzelesin; cedefingol; celecoxib (COX-2 inhibitor); chlorambucil; cirolemycin; cisplatin; cladribine; crisnatol mesylate; cyclophosphamide; cytarabine; dacarbazine; dactinomycin; daunorubicin hydrochloride; decitabine; dexormaplatin; dezaguanine; dezaguanine mesylate; diaziquone; docetaxel; doxorubicin; doxorubicin hydrochloride; droloxifene; droloxifene citrate; dromostanolone propionate; duazomycin; edatrexate; eflomithine hydrochloride; elsamitrucin; enloplatin; enpromate; epipropidine; epirubicin hydrochloride; erbulozole; esorubicin hydrochloride; estramustine; estramustine phosphate sodium; etanidazole; etoposide; etoposide phosphate; etoprine; fadrozole hydrochloride; fazarabine; fenretinide; floxuridine; fludarabine phosphate; fluorouracil; fluorocitabine; fosquidone; fostriecin sodium; gemcitabine; gemcitabine hydrochloride; hydroxyurea; idarubicin hydrochloride; ifosfamide; ilmofosine; iproplatin; irinotecan; irinotecan hydrochloride; lanreotide acetate; letrozole; leuprolide acetate; liarozole hydrochloride; lometrexol sodium; lomustine; losoxantrone hydrochloride; masoprocol; maytansine; mechlorethamine hydrochloride; megestrol acetate; melengestrol acetate; melphalan; menogaril; mercaptopurine; methotrexate; methotrexate sodium; metoprine; meturedepa; mitindomide; mitocarcin; mitocromin; mitogillin; mitomalcin; mitomycin; mitosper; mitotane; mitoxantrone hydrochloride; mycophenolic acid; nocodazole; nogalamycin; ormaplatin; oxisuran; paclitaxel; pegaspargase; peliomycin; pentamustine; peplomycin sulfate; perfosfamide; pipobroman; piposulfan; piroxantrone hydrochloride; plicamycin; plomestane; porfimer sodium; porfiromycin; prednimustine; procarbazine hydrochloride; puromycin; puromycin hydrochloride; pyrazofurin; riboprine; safingol; safingol hydrochloride; semustine; simtrazene; sparfosate sodium; sparsomycin; spirogermanium hydrochloride; spiromustine; spiroplatin; streptonigrin; streptozocin; sulofenur; talisomycin; tecogalan sodium; taxotere; tegafur; teloxantrone hydrochloride; temoporfin; teniposide; teroxirone; testolactone; thiamiprine; thioguanine; thiotepa; tiazofurin; tirapazamine; toremifene citrate; trestolone acetate; triciribine phosphate; trimetrexate; trimetrexate glucuronate; triptorelin; tubulozole hydrochloride; uracil mustard; uredepa; vapreotide; verteporfin; vinblastine sulfate; vincristine sulfate; vindesine; vindesine sulfate; vinepidine sulfate; vinglycinate sulfate; vinleurosine sulfate; vinorelbine tartrate; vinrosidine sulfate; vinzolidine sulfate; vorozole; zeniplatin; zinostatin; zorubicin hydrochloride; 20-epi-1,25 dihydroxyvitamin D3; 5-ethynyluracil; abiraterone; aclarubicin; acylfulvene; adecypenol; adozelesin; aldesleukin; ALL-TK antagonists; altretamine; ambamustine; amidox; amifostine; aminolevulinic acid; amrubicin; amsacrine; anagrelide; anastrozole; andrographolide; angiogenesis inhibitors; antagonist D; antagonist G; antarelix; anti-dorsalizing morphogenetic protein-1; antiandrogen, prostatic carcinoma; antiestrogen; antineoplaston; antisense oligonucleotides; aphidicolin glycinate; apoptosis gene modulators; apoptosis regulators; apurinic acid; ara-CDP-DL-PTBA; arginine deaminase; asulacrine; atamestane; atrimustine; axinastatin 1; axinastatin 2; axinastatin 3; azasetron; azatoxin; azatyrosine; baccatin III derivatives; balanol; batimastat; BCR/ABL antagonists; benzochlorins; benzoylstaurosporine; beta lactam derivatives; beta-alethine; betaclamycin B; betulinic acid; bFGF inhibitor; bicalutamide; bisantrene; bisaziridinylspermine; bisnafide; bistratene A; bizelesin; breflate; bropirimine; budotitane; buthionine sulfoximine; calcipotriol; calphostin C; camptothecin derivatives; capecitabine; carboxamide-amino-triazole; carboxyamidotriazole; CaRest M3; CARN 700; cartilage derived inhibitor; carzelesin; casein kinase inhibitors (ICOS); castanospermine; cecropin B; cetrorelix; chlorlns; chloroquinoxaline sulfonamide; cicaprost; cis-porphyrin; cladribine; clomifene analogues; clotrimazole; collismycin A; collismycin B; combretastatin A4; combretastatin analogue; conagenin; crambescidin 816; crisnatol; cryptophycin 8; cryptophycin A derivatives; curacin A; cyclopentanthraquinones; cycloplatam; cypemycin; cytarabine ocfosfate; cytolytic factor; cytostatin; dacliximab; decitabine; dehydrodidenmin B; deslorelin; dexamethasone; dexifosfamide; dexrazoxane; dexverapamil; diaziquone: didemnin B; didox; diethylnorspermine; dihydro-5-azacytidine; dihydrotaxol, 9-; dioxamycin; diphenyl spiromustine; docetaxel; docosanol; dolasetron; doxifluridine; doxorubicin; droloxifene; dronabinol; duocarmycin SA; ebselen; ecomustine; edelfosine; edrecolomab; eflomithine; elemene; emitefur; epirubicin; epristeride; estramustine analogue; estrogen agonists; estrogen antagonists; etanidazole; etoposide phosphate; exemestane; fadrozole; fazarabine; fenretinide; filgrastim; finasteride; flavopiridol; flezelastine; fluasterone; fludarabine; fluorodaunorunicin hydrochloride; forfenimex; formestane; fostriecin; fotemustine; gadolinium texaphyrin; gallium nitrate; galocitabine; ganirelix; gelatinase inhibitors; gemcitabine; glutathione inhibitors; hepsulfam; heregulin; hexamethylene bisacetamide; hypericin; ibandronic acid; idarubicin; idoxifene; idramantone; ilmofosine; ilomastat; imatinib (*e.g*., GLEEVEC^{®}), imiquimod; immunostimulant peptides; insulin-like growth factor-1 receptor inhibitor; interferon agonists; interferons; interleukins; iobenguane; iododoxorubicin; ipomeanol, 4-; iroplact; irsogladine; isobengazole; isohomohalicondrin B; itasetron; jasplakinolide; kahalalide F; lamellarin-N triacetate; lanreotide; leinamycin; lenograstim; lentinan sulfate; leptolstatin; letrozole; leukemia inhibiting factor; leukocyte alpha interferon; leuprolide+estrogen+progesterone; leuprorelin; levamisole; liarozole; linear polyamine analogue; lipophilic disaccharide peptide; lipophilic platinum compounds; lissoclinamide 7; lobaplatin; lombricine; lometrexol; lonidamine; losoxantrone; loxoribine; lurtotecan; lutetium texaphyrin; lysofylline; lytic peptides; maitansine; mannostatin A; marimastat; masoprocol; maspin; matrilysin inhibitors; matrix metalloproteinase inhibitors; menogaril; merbarone; meterelin; methioninase; metoclopramide; MIF inhibitor; mifepristone; miltefosine; mirimostim; mitoguazone; mitolactol; mitomycin analogues; mitonafide; mitotoxin fibroblast growth factor-saporin; mitoxantrone; mofarotene; molgramostim; Erbitux, human chorionic gonadotrophin; monophosphoryl lipid A+myobacterium cell wall sk; mopidamol; mustard anticancer agent; mycaperoxide B; mycobacterial cell wall extract; myriaporone; N-acetyldinaline; N-substituted benzamides; nafarelin; nagrestip; naloxone+pentazocine; napavin; naphterpin; nartograstim; nedaplatin; nemorubicin: neridronic acid; nilutamide; nisamycin; nitric oxide modulators; nitroxide antioxidant; nitrullyn; oblimersen (GENASENSE^{®}); O.sup.6-benzylguanine; octreotide; okicenone; oligonucleotides; onapristone; ondansetron; ondansetron; oracin; oral cytokine inducer; ormaplatin; osaterone; oxaliplatin; oxaunomycin; paclitaxel; paclitaxel analogues; paclitaxel derivatives; palauamine; palmitoylrhizoxin; pamidronic acid; panaxytriol; panomifene; parabactin; pazelliptine; pegaspargase; peldesine; pentosan polysulfate sodium; pentostatin; pentrozole; perflubron; perfosfamide; perillyl alcohol; phenazinomycin; phenylacetate; phosphatase inhibitors; picibanil; pilocarpine hydrochloride; pirarubicin; piritrexim; placetin A; placetin B; plasminogen activator inhibitor; platinum complex; platinum compounds; platinum-triamine complex; porfimer sodium; porfiromycin; prednisone; propyl bis-acridone; prostaglandin J2; proteasome inhibitors; protein A-based immune modulator; protein kinase C inhibitor; protein kinase C inhibitors, microalgal; protein tyrosine phosphatase inhibitors; purine nucleoside phosphorylase inhibitors; purpurins; pyrazoloacridine; pyridoxylated hemoglobin polyoxyethylene conjugate; raf antagonists; raltitrexed; ramosetron; ras farnesyl protein transferase inhibitors; ras inhibitors; ras-GAP inhibitor; retelliptine demethylated; rhenium Re 186 etidronate; rhizoxin; ribozymes; RII retinamide; rohitukine; romurtide; roquinimex; rubiginone B1; ruboxyl; safingol; saintopin; SarCNU; sarcophytol A; sargramostim; Sdi 1 mimetics; semustine; senescence derived inhibitor 1; sense oligonucleotides; signal transduction inhibitors; sizofuran; sobuzoxane; sodium borocaptate; sodium phenylacetate; solverol; somatomedin binding protein; sonermin; sparfosic acid; spicamycin D; spiromustine; splenopentin; spongistatin 1; squalamine; stipiamide; stromelysin inhibitors; sulfinosine; superactive vasoactive intestinal peptide antagonist; suradista; suramin; swainsonine; tallimustine; tamoxifen methiodide; tauromustine; tazarotene; tecogalan sodium; tegafur; tellurapyrylium; telomerase inhibitors; temoporfin; teniposide; tetrachlorodecaoxide; tetrazomine; thaliblastine; thiocoraline; thrombopoietin; thrombopoietin mimetic; thymalfasin; thymopoietin receptor agonist; thymotrinan; thyroid stimulating hormone; tin ethyl etiopurpurin; tirapazamine; titanocene bichloride; topsentin; toremifene; translation inhibitors; tretinoin; triacetyluridine; triciribine; trimetrexate; triptorelin; tropisetron; turosteride; tyrosine kinase inhibitors; tyrphostins; UBC inhibitors; ubenimex; urogenital sinus-derived growth inhibitory factor; urokinase receptor antagonists; vapreotide; variolin B; velaresol; veramine; verdins; verteporfin; vinorelbine; vinxaltine; vitaxin; vorozole; zanoterone; zeniplatin; zilascorb; and zinostatin stimalamer., or any analog or derivative variant of the foregoing. In specific embodiments, chemotherapy is employed in conjunction with the disclosure, for example before, during and/or after administration of the disclosure. Exemplary chemotherapeutic agents include at least dacarbazine (also termed DTIC), temozolimide, paclitaxel, cisplatin, carmustine, fotemustine, vindesine, vincristine, or bleomycin.

### B. Radiotherapy

Other factors that cause DNA damage and have been used extensively include what are commonly known as γ-rays, X-rays, and/or the directed delivery of radioisotopes to tumor cells. Other forms of DNA damaging factors are also contemplated such as microwaves and UV-irradiation. It is most likely that all of these factors effect a broad range of damage on DNA, on the precursors of DNA, on the replication and repair of DNA, and on the assembly and maintenance of chromosomes. Dosage ranges for X-rays range from daily doses of 50 to 200 roentgens for prolonged periods of time (3 to 4 wk), to single doses of 2000 to 6000 roentgens. Dosage ranges for radioisotopes vary widely, and depend on the half-life of the isotope, the strength and type of radiation emitted, and the uptake by the neoplastic cells.

The terms "contacted" and "exposed," when applied to a cell, are used herein to describe the process by which a therapeutic construct and a chemotherapeutic or radiotherapeutic agent are delivered to a target cell or are placed in direct juxtaposition with the target cell. To achieve cell killing or stasis, both agents are delivered to a cell in a combined amount effective to kill the cell or prevent it from dividing.

### C. Immunotherapy

Immunotherapeutics generally rely on the use of immune effector cells and molecules to target and destroy cancer cells. The immune effector may be, for example, an antibody specific for some marker on the surface of a tumor cell. The antibody alone may serve as an effector of therapy or it may recruit other cells to actually effect cell killing. The antibody also may be conjugated to a drug or toxin (chemotherapeutic, radionuclide, ricin A chain, cholera toxin, pertussis toxin, *etc*.) and serve merely as a targeting agent. Alternatively, the effector may be a lymphocyte carrying a surface molecule that interacts, either directly or indirectly, with a tumor cell target. Various effector cells include cytotoxic T cells and NK cells.

Immunotherapy could thus be used as part of a combined therapy, in conjunction with the present cell therapy. The general approach for combined therapy is discussed below. Generally, the tumor cell must bear some marker that is amenable to targeting, *i.e.,* is not present on the majority of other cells. Many tumor markers exist and any of these may be suitable for targeting in the context of the present disclosure. Common tumor markers include carcinoembryonic antigen, prostate specific antigen, urinary tumor associated antigen, fetal antigen, tyrosinase (p97), gp68, TAG-72, HMFG, Sialyl Lewis Antigen, MucA, MucB, PLAP, estrogen receptor, laminin receptor, erb B and p155, and the like.

Immunotherapy may include interleukin-2 (IL-2) or interferon (IFN), for example. In certain embodiments, the immunotherapy is an antibody against a Notch pathway ligand or receptor, *e.g.*, an antibody against DLL4, Notch1, Notch2/3, Fzd7, or Wnt. In certain other embodiments, the immunotherapy is an antibody against r-spondin (RSPO) 1, RSPO2, RSPO3 or RSPO4.

### D. Genes

In yet another embodiment, the secondary treatment is a gene therapy in which a therapeutic polynucleotide is administered before, after, or at the same time as the clinical embodiments of the present disclosure. A variety of expression products are encompassed within the disclosure, including inducers of cellular proliferation, inhibitors of cellular proliferation, or regulators of programmed cell death.

### E. Surgery

Approximately 60% of persons with cancer will undergo surgery of some type, which includes preventative, diagnostic or staging, curative and palliative surgery. Curative surgery is a cancer treatment that may be used in conjunction with other therapies, such as the treatment of the present disclosure, chemotherapy, radiotherapy, hormonal therapy, gene therapy, immunotherapy and/or alternative therapies.

Curative surgery includes resection in which all or part of cancerous tissue is physically removed, excised, and/or destroyed. Tumor resection refers to physical removal of at least part of a tumor. In addition to tumor resection, treatment by surgery includes laser surgery, cryosurgery, electrosurgery, and miscopically controlled surgery (Mohs' surgery). It is further contemplated that the present disclosure may be used in conjunction with removal of superficial cancers, precancers, or incidental amounts of normal tissue.

Upon excision of part of all of cancerous cells, tissue, or tumor, a cavity may be formed in the body. Treatment may be accomplished by perfusion, direct injection or local application of the area with an additional anti-cancer therapy. Such treatment may be repeated, for example, every 1, 2, 3, 4, 5, 6, or 7 days, or every 1, 2, 3, 4, and 5 weeks or every 1, 2, 3, 4, 5, 6, 7, 8, 9, 10, 11, or 12 months. These treatments may be of varying dosages as well.

### F. Other agents

It is contemplated that other agents may be used in combination with the present disclosure to improve the therapeutic efficacy of treatment. These additional agents include immunomodulatory agents, agents that affect the upregulation of cell surface receptors and GAP junctions, cytostatic and differentiation agents, inhibitors of cell adhesion, or agents that increase the sensitivity of the hyperproliferative cells to apoptotic inducers. Immunomodulatory agents include tumor necrosis factor; interferon alpha, beta, and gamma; IL-2 and other cytokines; F42K and other cytokine analogs; or MIP-1, MIP-1beta, MCP-1, RANTES, and other chemokines. It is further contemplated that the upregulation of cell surface receptors or their ligands such as Fas / Fas ligand, DR4 or DR5 / TRAIL would potentiate the apoptotic inducing abililties of the present disclosure by establishment of an autocrine or paracrine effect on hyperproliferative cells. Increased intercellular signaling by elevation of the number of GAP junctions would increase the anti-hyperproliferative effects on the neighboring hyperproliferative cell population. In other embodiments, cytostatic or differentiation agents can be used in combination with the present disclosure to improve the anti-hyerproliferative efficacy of the treatments. Inhibitors of cell adhesion are contemplated to improve the efficacy of the present disclosure. Examples of cell adhesion inhibitors are focal adhesion kinase (FAKs) inhibitors and Lovastatin. It is further contemplated that other agents that increase the sensitivity of a hyperproliferative cell to apoptosis, such as the antibody c225, could be used in combination with the present disclosure to improve the treatment efficacy.

### EXAMPLES

The following examples are included to demonstrate preferred aspects of the disclosure. It should be appreciated by those of skill in the art that the techniques disclosed in the examples that follow represent techniques discovered by the inventor to function well in the practice of the disclosure, and thus can be considered to constitute preferred modes for its practice. However, those of skill in the art should, in light of the present disclosure, appreciate that many changes can be made in the specific embodiments which are disclosed and still obtain a like or similar result.

### EXAMPLE 1

### TRIPARTITE SIGNALING IMMUNE CELLS

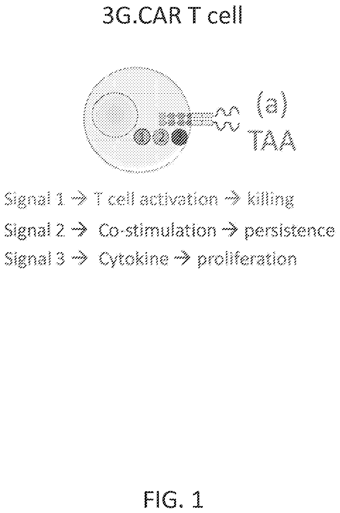

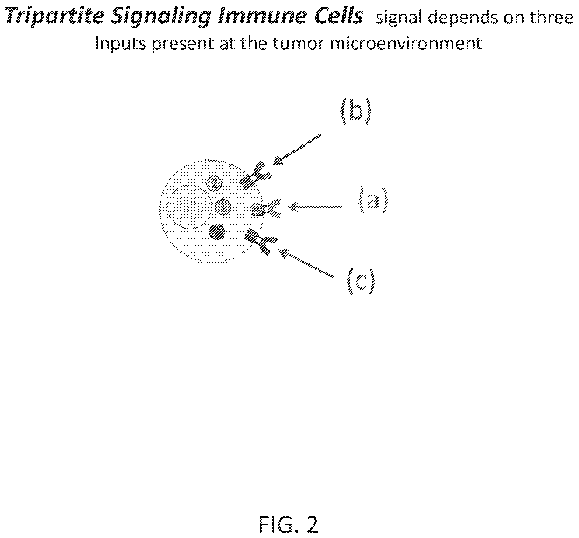

In one embodiment, cells that recognize a combination of three signals present at the tumor microenvironment (for example) are provided. Embodiments encompass the generation of an immune cell whereby signals for antigen stimulation, co-stimulation, and cytokine signals are separated into three different molecules (such as receptors) rather than incorporating all signals together into a single molecule (such as a receptor), for example as in 3^{rd} generation CARs ("3G.CARs"; FIG. 1). Therefore, in contrast to the 3^{rd} generation CARs where the sole presence of the antigen results in the activation of these three different signal pathways, in embodiments contemplated herein the immune cells are fully activated (signal I, II and III) only in the presence of a particular molecular signature present only at a certain location, such as a tumor microenvironment (FIG. 2). Such an embodiment increases the specificity and safety of the immunotherapy.

To overcome the toxicity issues of later generation CARs while still maintaining the potency of an immune cell response, embodiments of the disclosure provide cells that express three separate receptors comprising the endodomains responsible for signals 1, 2 and 3 individually. By this unique strategy, the immune cells will require three input signals in order to activate the three individual receptors resulting in the potent activation of the immune cells (FIG. 2).

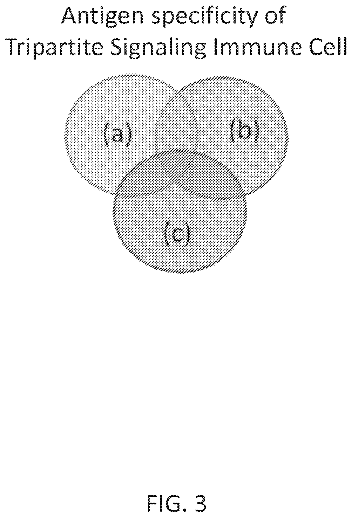

Through the judicious selection of input signals (a), (b) and (c) that are specifically present at the tumor microenvironment, one can control the immune cells to undergo robust activation at the tumor site where all three signals are present, and because of the low probability that all three signals would co-exist in a normal tissue in the body different than the tumor, the engineered immune cells are highly specific (FIG. 3).

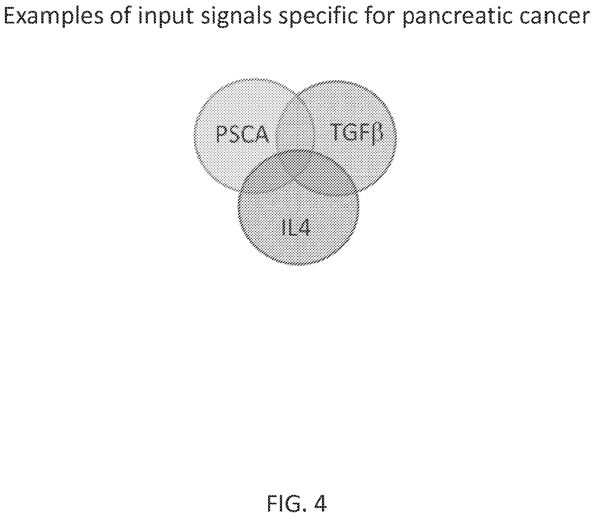

In one embodiment, an immune cell for the treatment of cancer is provided. Any cancer may be treated with immune cells contemplated herein. In specific embodiments, pancreatic cancer is treated with immune cells. It has been shown that prostate stem cell antigen (PSCA) has been overexpressed in pancreatic tumors with limited expression in normal tissues making it an ideal target antigen and input signal (a). In addition, inhibitory cytokines such as IL4 and TGFβ have also been shown to be present at elevated levels in pancreatic cancer patients, making them ideal candidates for input signal (b) and (c). Hence, PSCA (a), IL4 (b) and TGFβ (c) serve as an example of three input signals that exclusively intersect at the pancreatic tumor microenvironment (FIG. 4).

To utilize molecular signatures present at the tumor microenvironment, immune cells of the disclosure are genetically modified to express receptors that can recognize these inputs and transmit downstream signals that can result in potent activation. In this way the expression of PSCA (as an example) will transmit signal (I), the presence of TGFβ will induce signal (II) while the presence of IL4 cytokine will provide signal (III). In this way, the immune cells are activated in the presence of these three signals only at the tumor site. Such methods provide a much more specific therapy when compared with the current state of the art, such as with third generation CARs.

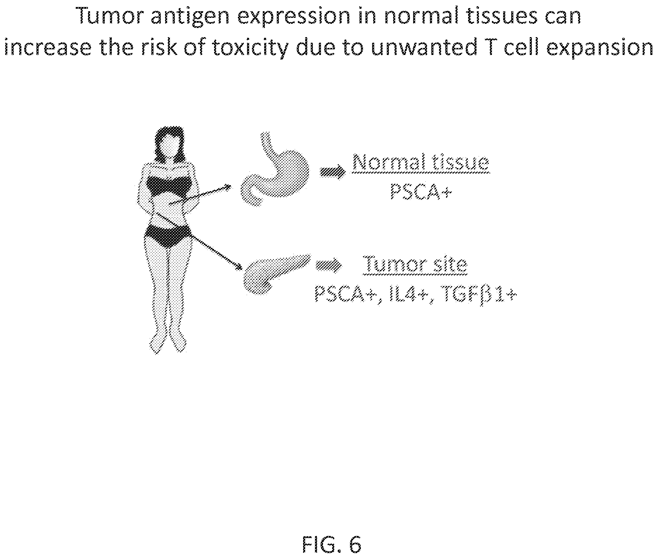

In order to compare 3G.CAR and tripartite signaling cells of the disclosure in their ability to expand and persist at the tumor site where all three signals co-exist vs. normal tissues such as the stomach that express low levels of PSCA and hence, could be potentially targeted resulting in undesirable side effects, pancreatic cancer was used as a model system because of the expression of PSCA, IL4 and TGFβ (FIG. 6).

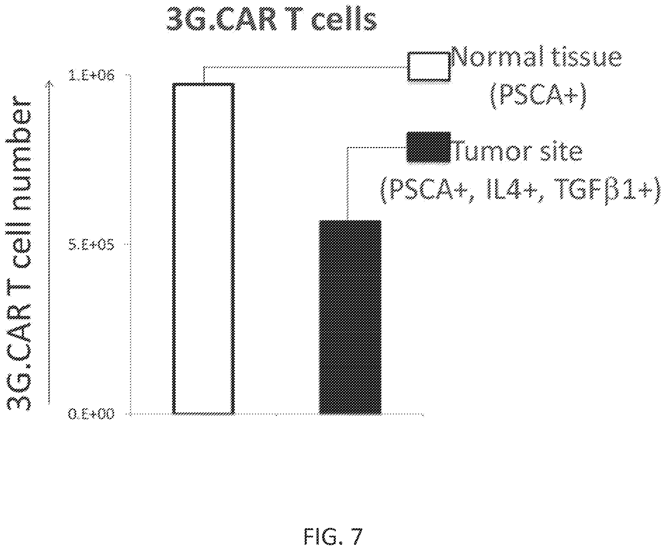

The expansion of 3G CAR T cells when challenged with cell lines mimicking normal tissue expressing only PSCA, resulted in a profound expansion of 3G.CAR T cells as illustrated in FIG. 7. In contrast, the expansion of 3G.CAR T cells in conditions that recapitulate the tumor microenvironment was significantly reduced. This suggests that the T cells modified with 3G.CAR can preferentially expand at normal tissue expressing the target antigen leading to potential toxicity.

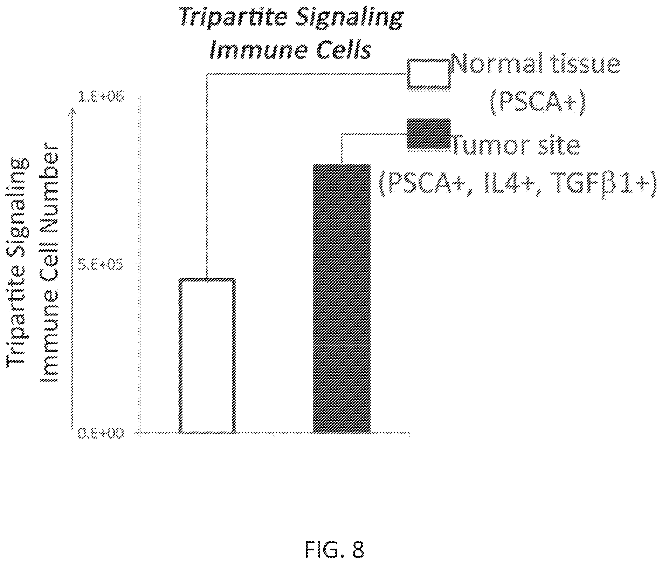

The expansion of the tripartite signaling cells was evaluated. (FIG. 8) Different from the 3G.CAR cells, tripartite signaling cells exhibit a preferential expansion in conditions that mimic the tumor signature (PSCA, IL4, TGFβ). Importantly, tripartite signaling cells demonstrated a reduced expansion when challenged with normal tissue that expresses the antigen only. These results suggest that tripartite signaling cells can elicit a potent response at the tumor site with minimal activity at normal tissue sites, highlighting the potency and safety of this approach.

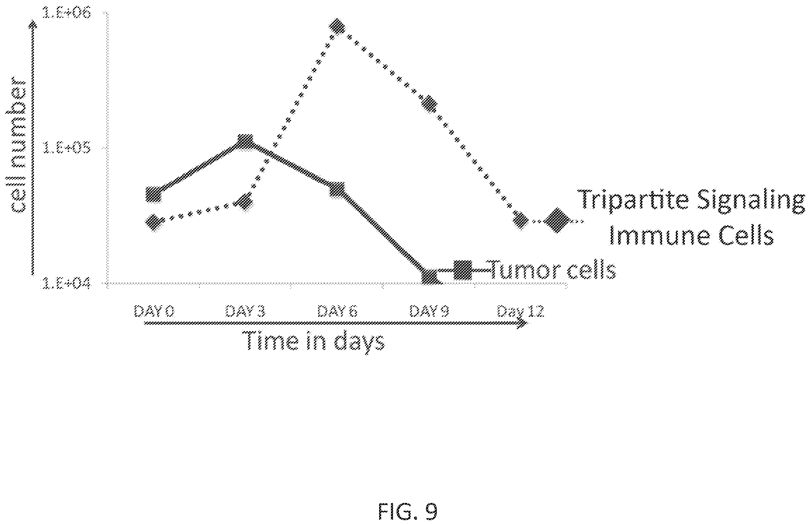

Next, the potency of the tripartite signaling cells cultured with cells mimicking the pancreatic tumor microenvironment (PSCA, IL4, TGFβ) was evaluated. As shown in FIG. 9, there was an initial expansion of the cells, which resulted in complete elimination of tumor cells by day 9. Importantly, after the tumor was eliminated the cells rapidly contracted in numbers, suggesting that in absence of the tumor signature, the cells will cease to persist, demonstrating an additional safety feature of this modification.

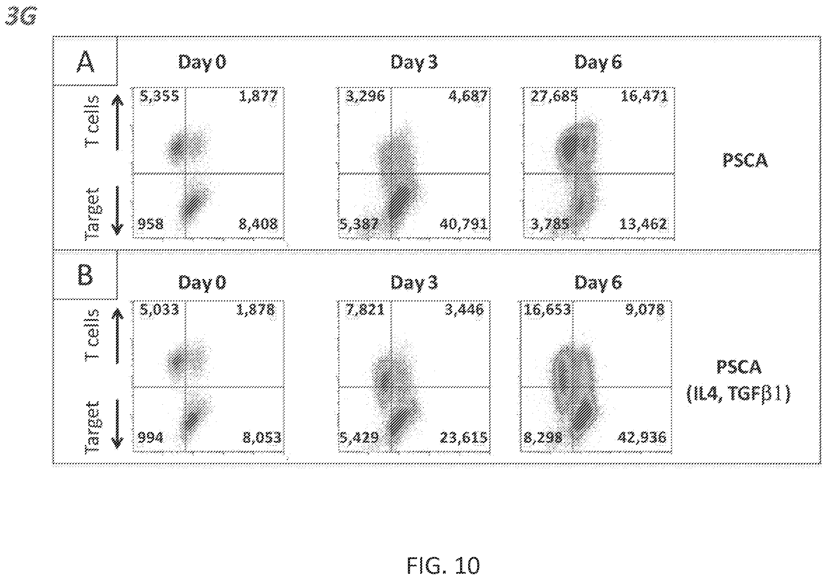

As illustrated in FIG. 10, T cells modified with 3G CAR demonstrated better expansion and cytolytic function when challenged only with the antigen (Panel A). In contrast, in conditions that mimic the tumor microenvironment, 3G.CAR T cells demonstrated a decrease in expansion and anti-tumor effect (Panel B).

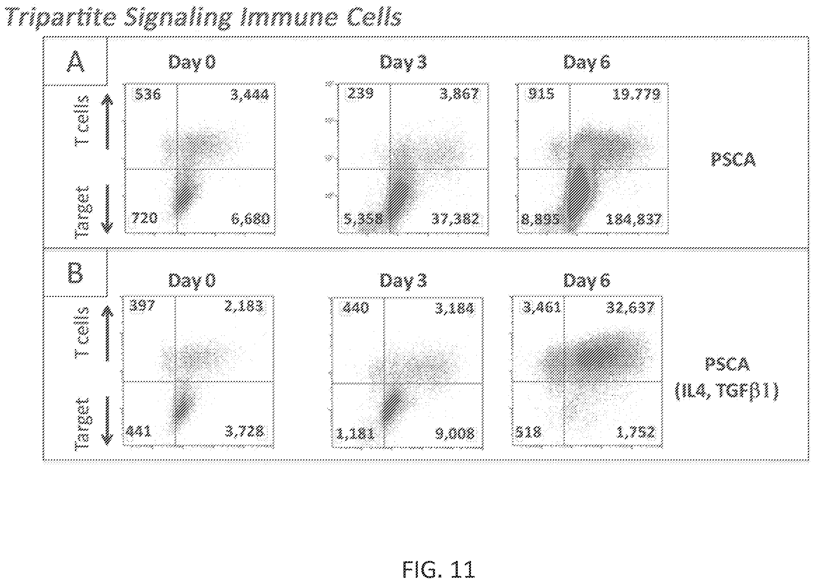

As illustrated in FIG. 11, tripartite signaling immune cells challenged with conditions that mimic the tumor microenvironment demonstrated a robust T cell expansion resulting in complete elimination of the tumor cells, while in contrast, when challenged with cells expressing only the antigen (normal tissue), tripartite signaling immune cells demonstrate a decreased cytolytic function because of the lack of persistence.

### EXAMPLE 2 (COMPARATIVE)

### BIPARTITE SIGNALING IMMUNE CELLS

In another aspect of the disclosure, cells that recognize a combination of only two signals present at the tumor microenvironment are provided. This can be accomplished by modifying immune cells to express two different molecules (such as receptors): (i) a molecule (such as a receptor) that recognizes a ligand (such as an antigen) and leads to TCR activation (for example, CD3z), with an example being a first generation CAR (or native or engineered T cell receptor) targeting a tumor antigen; and (ii) a receptor that recognizes a different ligand but transmits a cytokine signal (for example, IL7 endodomain), referred to in this application as bipartite signaling immune cells.

IL4 is present at elevated levels in primary pancreatic tumors. Logsdon and colleagues performed gene expression profiling on pancreatic adenocarcinoma and normal pancreas demonstrating elevated IL4 levels in pancreatic tumors (fold change 19.78, p = 0.001) (FIG. 31A). IHC studies scored based on IL4 expression intensity confirmed this observations (FIG. 31B). FIG. 31C illustrates elevated IL4 cytokine levels in patient serum (n=7), as quantitatively measured by ELISA. FIG. 31D illustrates the IL4 cytokine concentration collected from the serum of mice engrafted with a IL4 producing tumor, demonstrating IL4 cytokine concentrations of 6.6pg/ml IL4 in the periphery (similar to patient serum levels), while the tumor conc. was approx. 100x times (554.2/ml). Importantly, 4/7R-modified cells were able to expand only in IL4 levels predicted to be present at the tumor (FIG. 31E)).

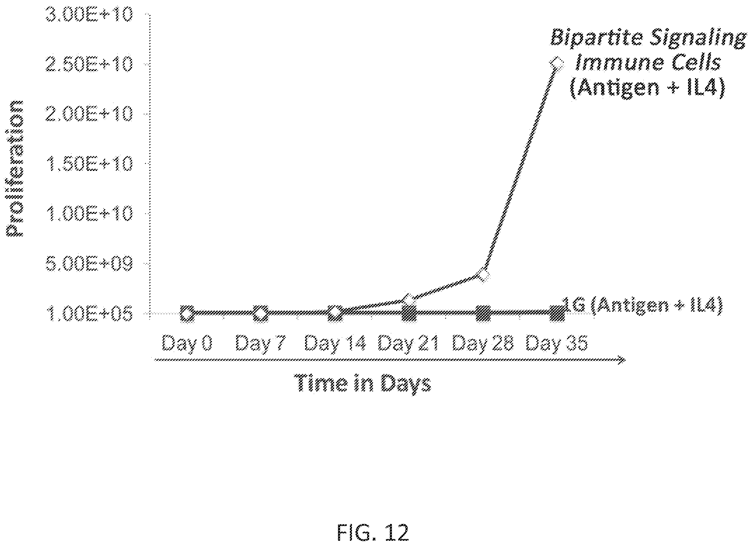

To assess whether bipartite signaling immune cells (such as T cells modified to express a first generation CAR.PSCA in combination with a receptor that transmits an IL7 receptor signal in response to the IL4 cytokine; said exemplary receptor may be referred to herein as 4/7R) exhibit a superior and selective expansion in presence of the antigen (PSCA) and cytokine (IL4), both present at the tumor microenvironment, the expansion of the immune cells *vs.* 1G.CAR PSCA was evaluated in response to weekly antigen and cytokine stimulation (PSCA and IL4). As shown in FIG. 12, only bipartite signaling immune cells were capable of expanding under these conditions.

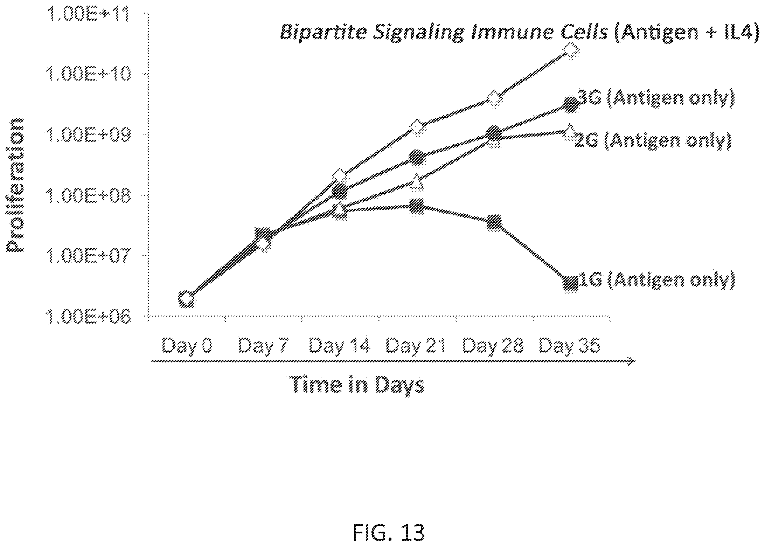

Next, the expansion of the bipartite signaling immune cells was compared to T cells modified with either second (2G) or third generation (3G) CAR. T cells modified with 2G and 3G CARs expand in presence of weekly antigen stimulation, as illustrated in FIG. 13. Importantly, bipartite signaling immune cells exhibit not only a superior expansion when compared to the 3G.CAR, but this was achieved only in the presence of antigen and cytokine stimulation.

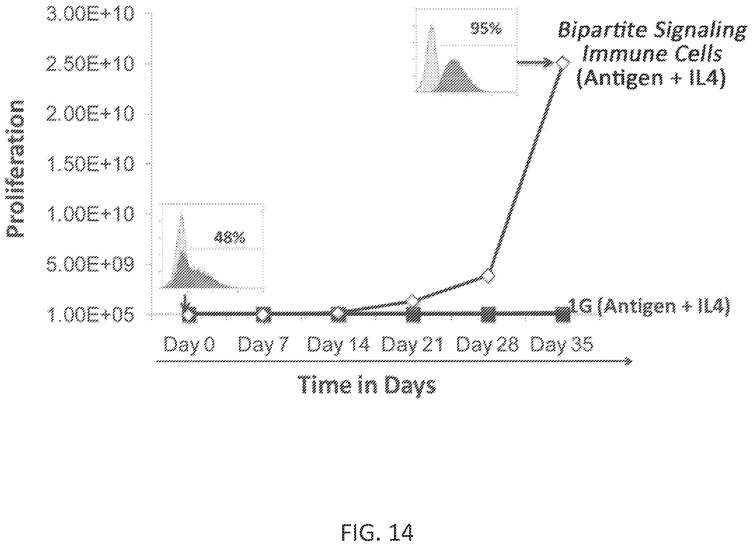

Importantly, bipartite signaling immune cells exhibit an enrichment for the transgene once cultured with the antigen and IL4 as demonstrated in FIG. 14, where the expression of the transgene increased from 48% to 95% in 5 weeks.

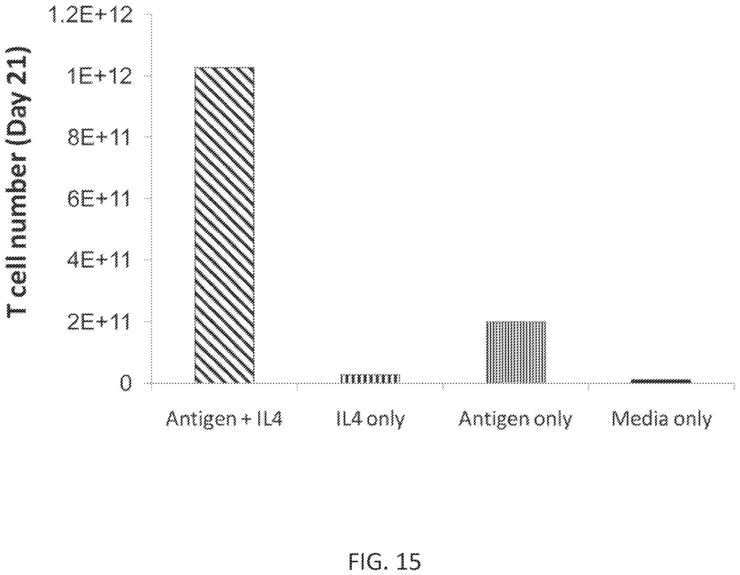

Next, to evaluate the safety profile of these bipartite signaling immune cells, cells were cultured in conditions where the antigen and cytokine were present together or individually. As illustrated in FIG. 15, bipartite signaling immune cells were able to expand only in response to the combination of antigen and cytokine. In contrast, bipartite signaling immune cells exhibit marginal expansion when either the antigen or cytokine were provided independently, suggesting that bipartite signaling immune cells have a proliferative advantage at the tumor site where both antigen and cytokine are present. Consequently, the approach is safer and more potent than conventional 3G CAR T cell therapy.

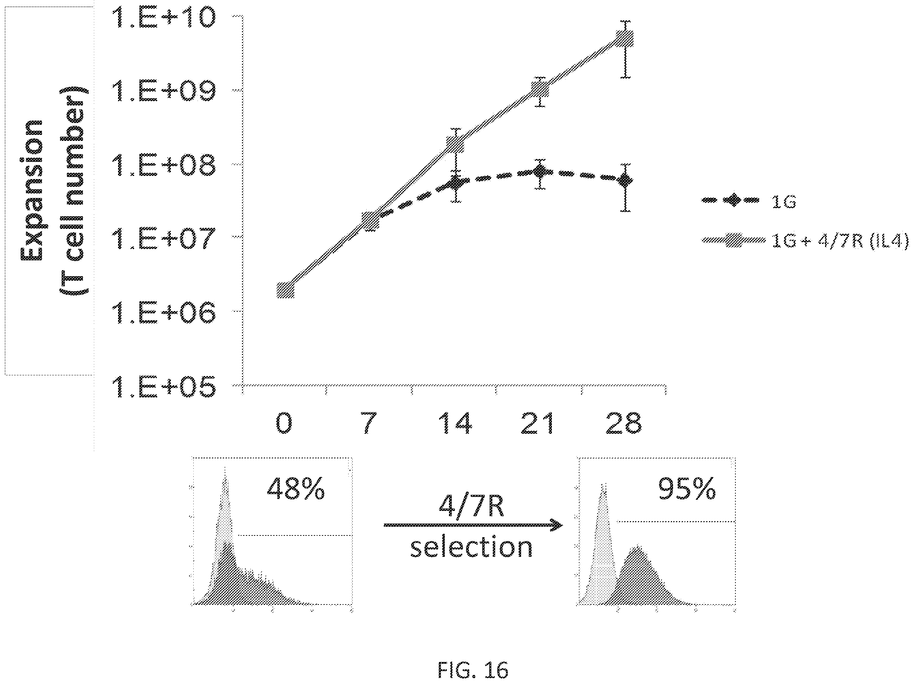

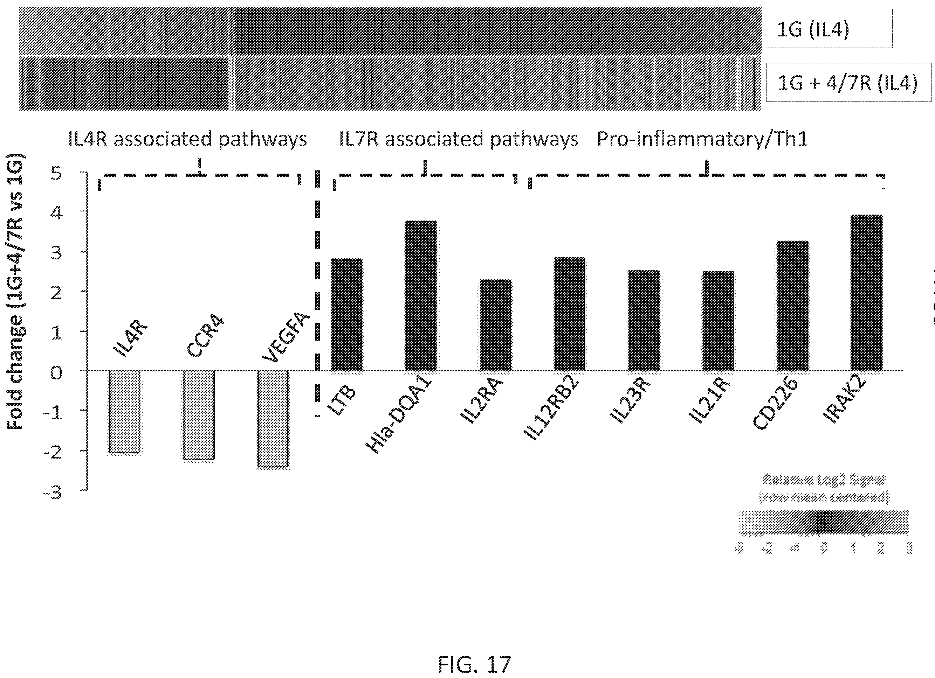

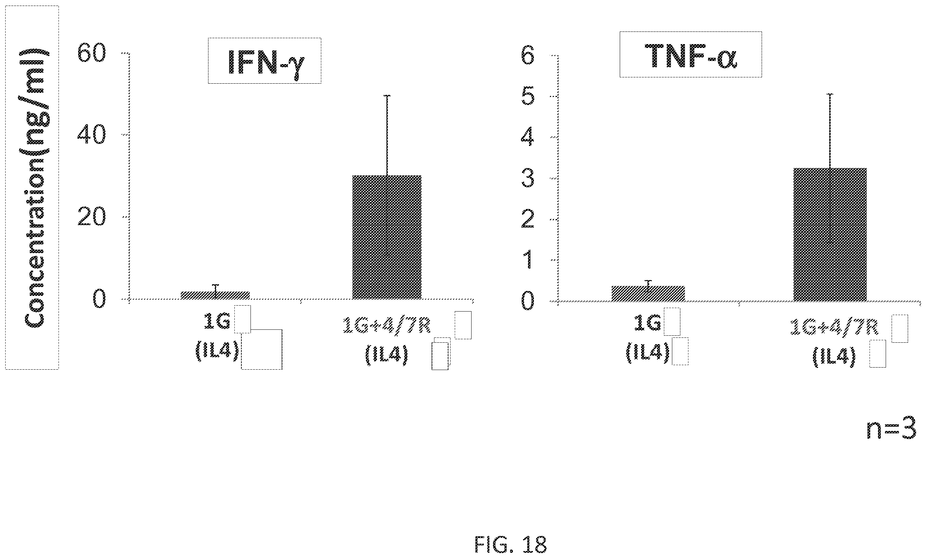

Further experiments were conducted to show the merits of employing chimeric cytokine receptors in immune cells. FIG. 16 shows that the exemplary 4/7R promotes proliferation of 1G CAR T cells in presence of IL4, and that in such cells the 4/7R altered the gene expression profile (FIG. 17) in the cells. FIG. 18 demonstrates that 4/7R retains a Th1 polarized cytokine expression profile in 1G T cells after exposure to IL4.

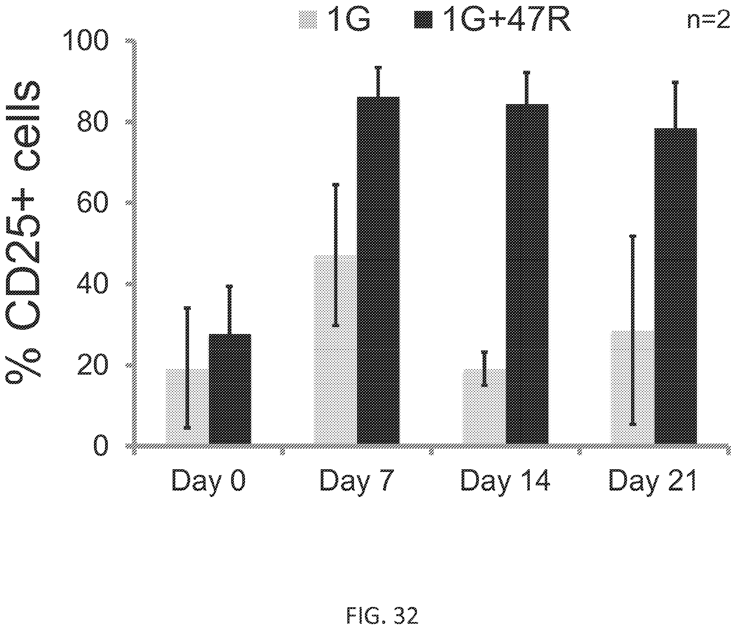

The presence of antigen and IL4 with bipartite T cells expressing 1G CAR and 4/7R results in upregulation of CD25, demonstrating the activation status of modified T cells (FIG. 32).

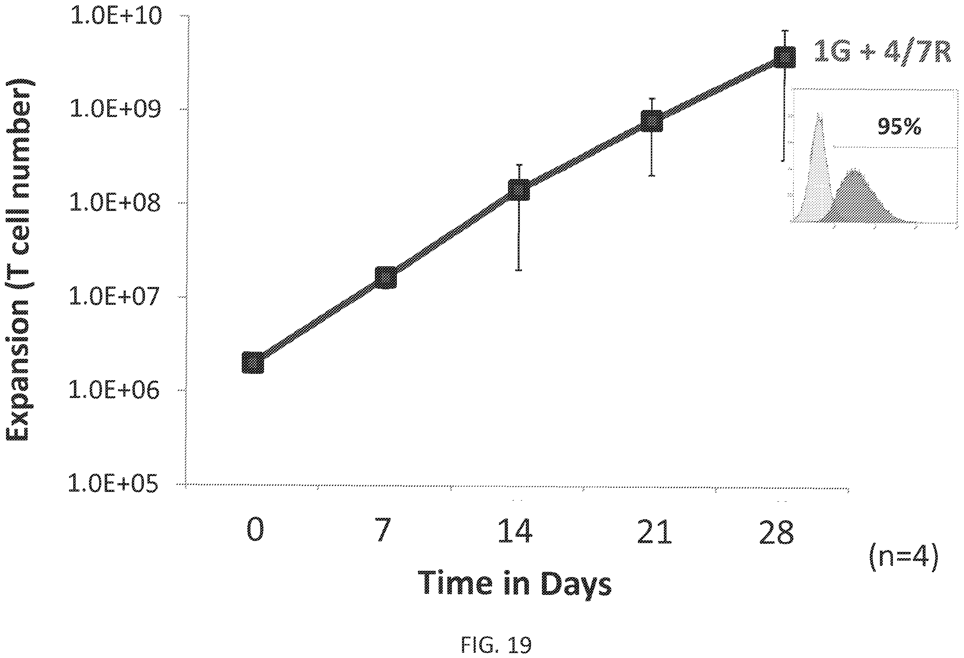

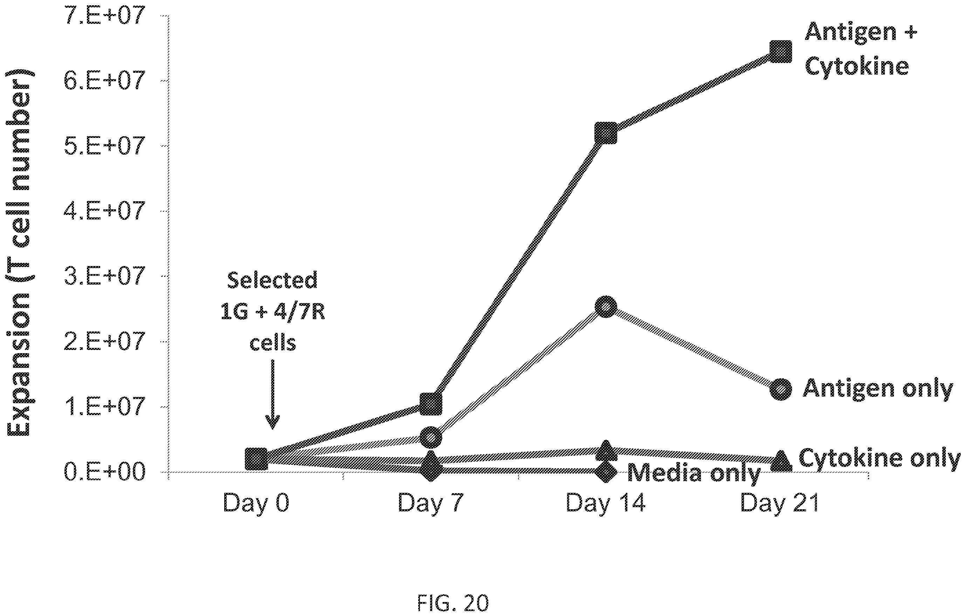

FIG. 19 demonstrates selective expansion due to 4/7R expression, and 1G + 4/7R T cells are antigen and cytokine dependent (FIG. 20).

Therefore, 4/7R can (i) protect and induce proliferation (ii) change in the gene expression profile and (iii) retain expression levels of prototypic Th1 polarizing cytokines in 4/7R CAR T cells exposed to IL4. Importantly, T cell growth and survival are dependent on the presence of both signals- antigen and cytokine.

### EXAMPLE 3

### CHIMERIC T-BBR RECEPTOR IN IMMUNE CELLS

In particular embodiments, immune cells comprise a chimeric cytokine receptor that includes an exodomain of native TGFβR and an endodomain of the co-stimulatory molecule 4-1BB ("T-BBR") . T-BBR expression in CAR PSCA T cells (for example) enables the cells to overcome TGFβ inhibition.

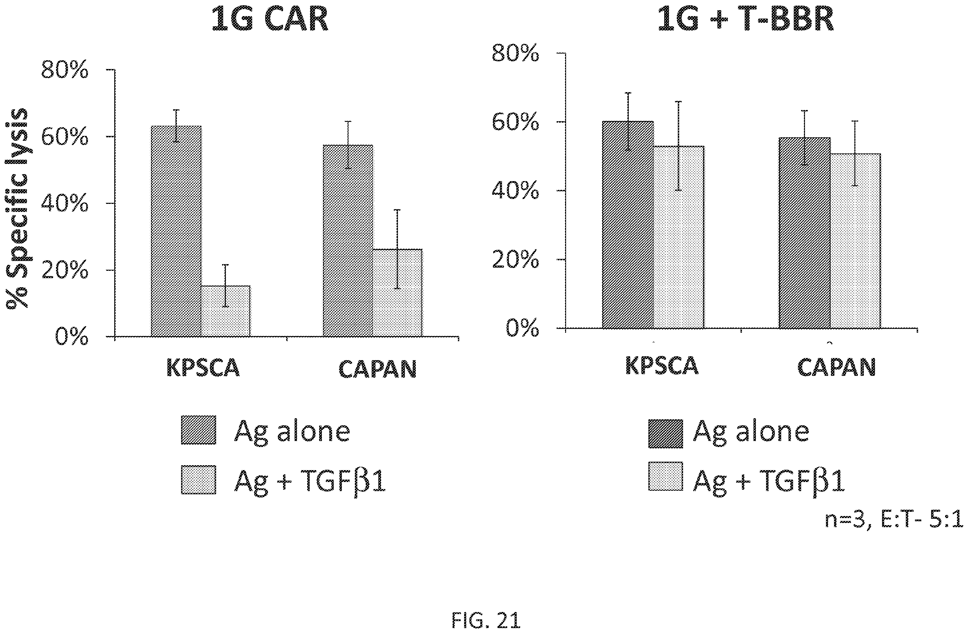

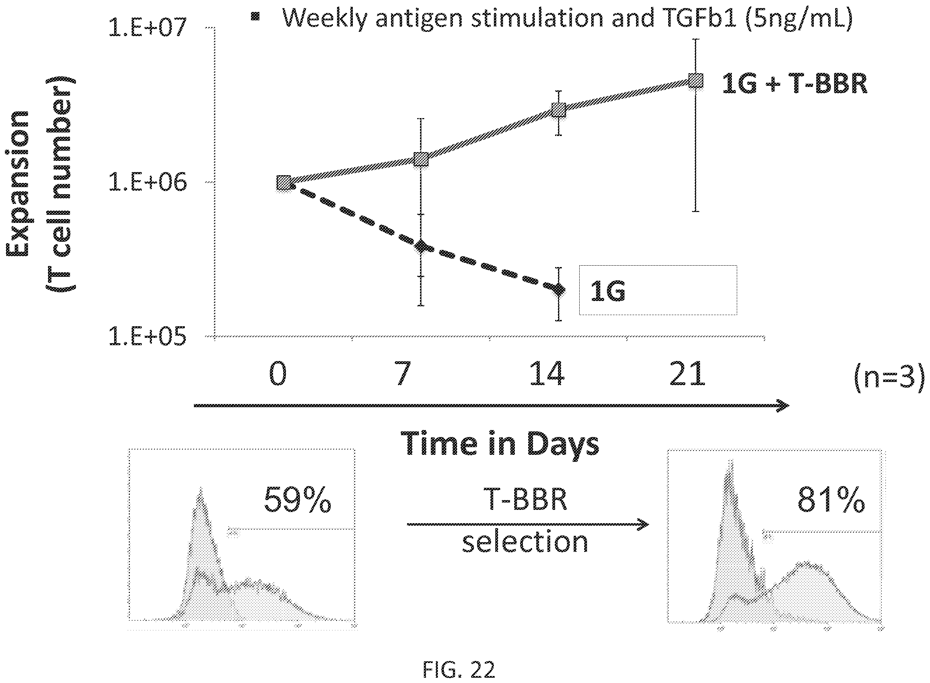

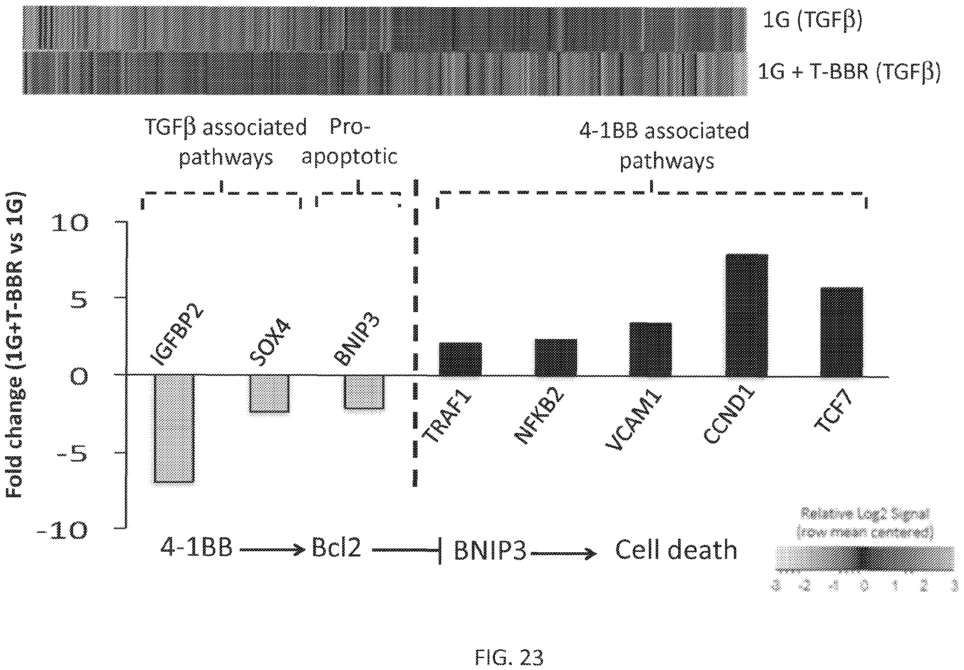

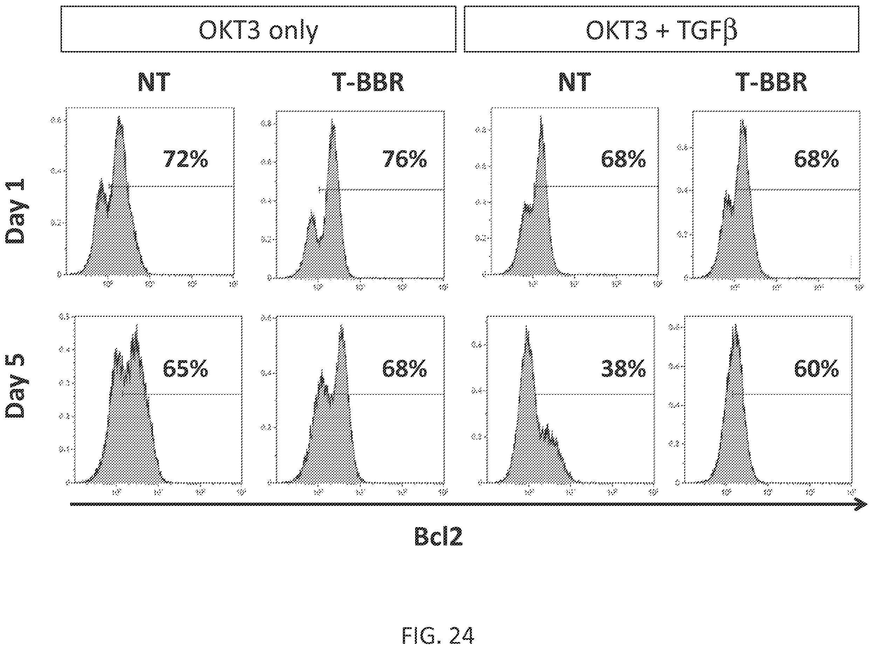

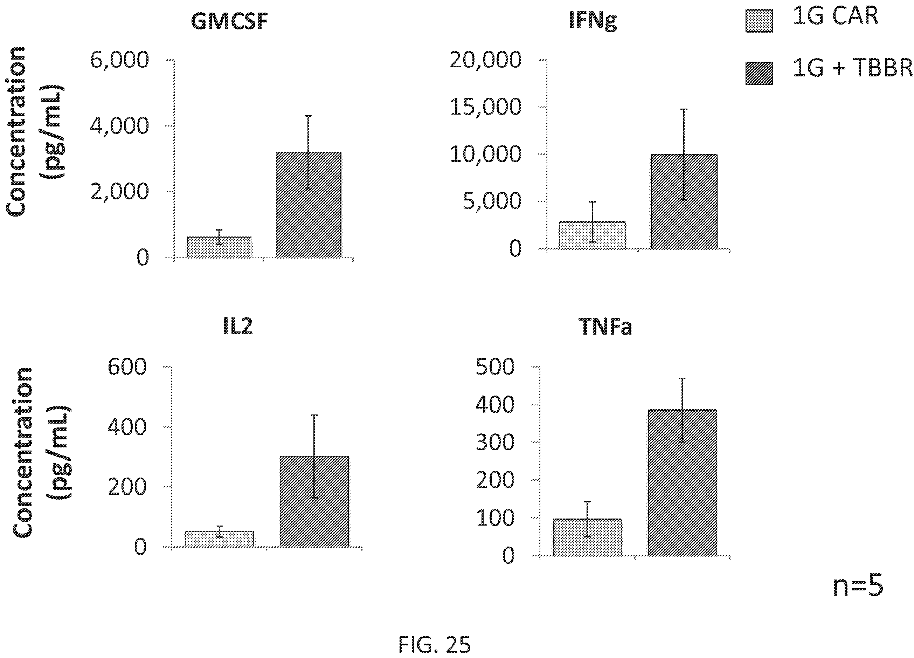

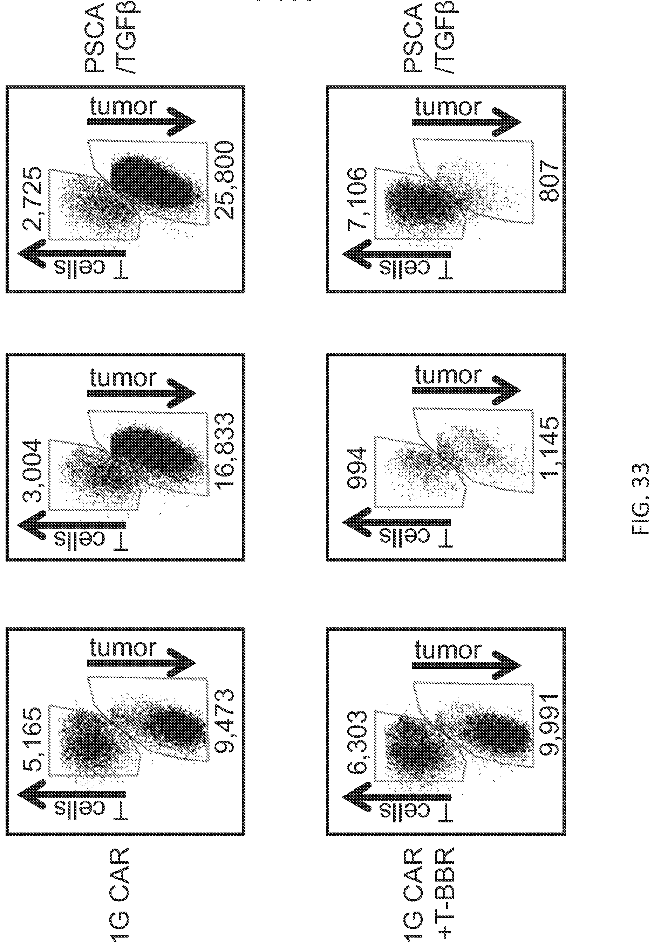

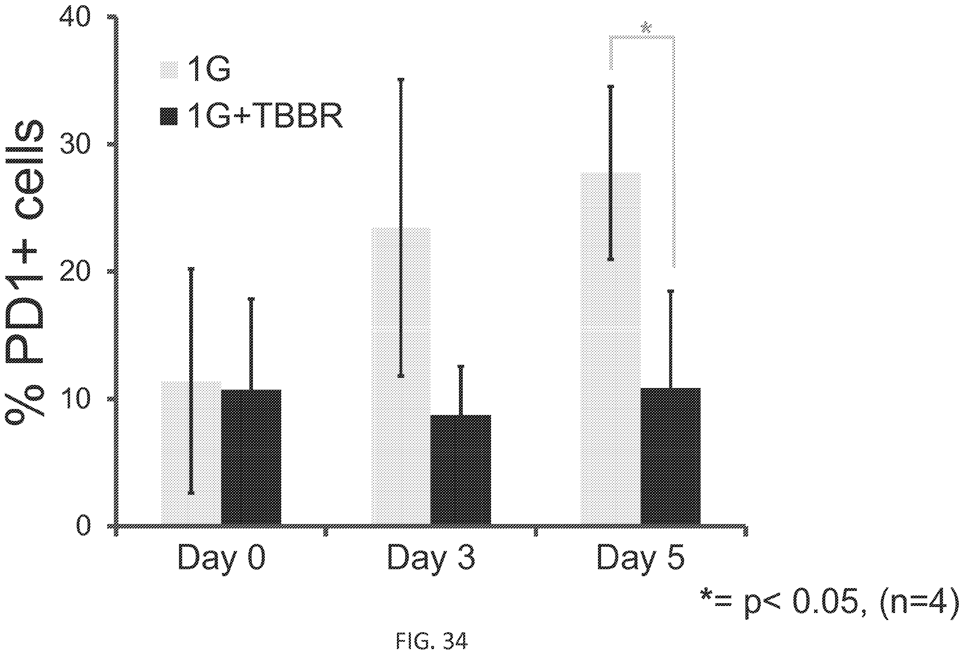

Modification of 1G CAR T cells to express T-BBR did not affect CAR function, and the cells maintain cytolytic function of CAR T cells exposed to TGFβ (FIG. 21). FIG. 22 shows that T-BBR protected CAR T cells exposed to TGFβ. The bipartite-modified T cells expressing 1G CAR and T-BBR were able to eliminate tumor cells in presence of TGFβ while in contrast, the administration of TGFβ inhibited the cytolytic function and prevented elimination of target cells by 1G CAR T cells alone (FIG. 33).Furthermore, T-BBR alters gene expression of 1G CAR T cells (FIG. 23), yet Bcl2 expression was maintained in T-BBR modified T cells (FIG. 24) as was a Th1 polarized response (FIG. 25) after exposure to TGFβ. Transgenic expression of T-BBR protected CAR T cells from T cell exhaustion measured by down-regulation of PD-1 expression following administration of TGFβ (FIG. 34).

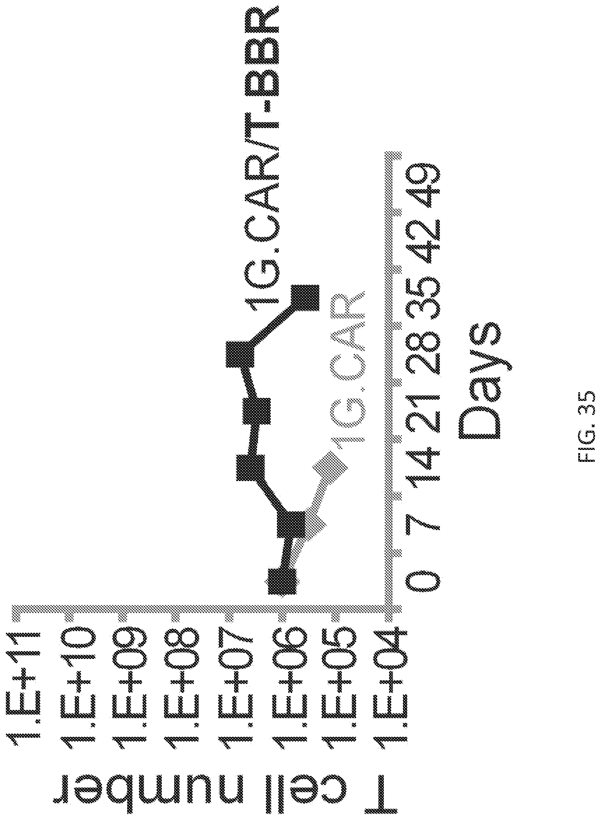

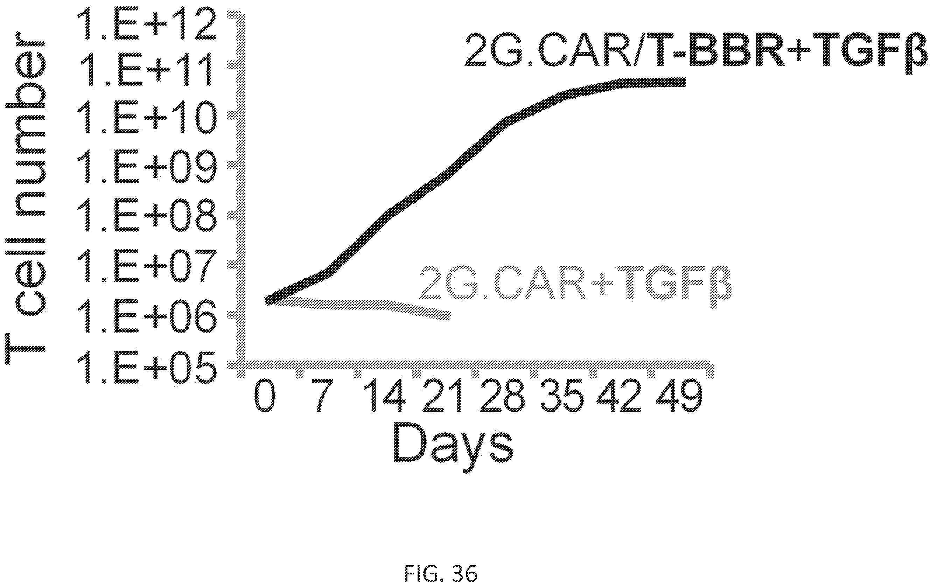

Double transgenic T cells expressing 1G CAR with T-BBR survive but do not expand as illustrated in (FIG. 35). In contrast, the bipartite T cells expressing T-BBR along with 2G CAR containing signals 1 and 2 resulted in profound T cell expansion in presence of TGFβ, supporting the requirement of signals 1, 2 and 3 for sustained T cell activity (FIG. 36).

Therefore, T-BBR did not affect cytolytic function of CAR T cells, it allows maintenance of the cytolytic activity and promotes the survival of 1G T cells exposed to TGFβ. T-BBR also altered the gene expression profile of CAR T cells cultured in TGFβ.

### EXAMPLE 4

### TRIPARTITE SIGNALING IMMUNE CELLS WITH EXEMPLARY CHIMERIC CYTOKINE RECEPTOR

Recent clinical success in the treatment of B cell malignancies using chimeric antigen receptors (CAR)-modified T cells has been directly linked with the ability of the infused cells to expand *in vivo* and persist long-term. However, while the inclusion of co-stimulatory endodomains has been shown to enhance CAR T cell potency the risk of toxicity is also increased due to the paucity of true tumor-specific target antigens leading to "on target, off tumor" effects. In one embodiment of the disclosure, T cell therapy is extended to solid tumors using an approach that overcomes two barriers; (i) the lack of unique target antigens, and (ii) the immunosuppressive tumor microenvironment, which can subvert the effector function and limit the persistence of infused cells.

T cells were engineered to express three independent recetpros to improve the tumor specificity and enhance the safety profile of the cells. When the receptors are simultaneously engaged with 3 different tumor ligands, signals that imitate natural T cell physiology are initiated: antigen recognition (signal 1), co-stimulation (signal 2), and cytokine (signal 3). In certain embodiments, these cells are referred to as SmarT cells. To ensure tumor selectivity, the receptors are customized to recognize a tumor-specific gene expression "pattern" rather than a single target antigen, thereby ensuring that cells become operative only in the tumor environment and not in normal tissues.

T cells were engineered to target pancreatic cancer by first identifying a tumor signature: prostrate stem cell antigen (PSCA), II,4 and TGFβ1. Second, a 1G CAR was generated to reliably generate an antigen recognition signal against PSCA, which allowed modified T cells to specifically kill PSCA+ (CAPAN1 and K562-PSCA) and not PSCA- targets (293T) (74±4%, 73±6% and 9±3% specific lysis, respectively, 10:1 E:T, n=3). Next, the CAR-PSCA T cells were further modified to stimulate cytokine signaling by expressing a chimeric cytokine receptor (CCR) "4/7R" that fuses the exodomain of the IL4R to the endodomain of the II,7R (30-72% double-positive cells; n=4). As expected, chronic exposure to IL4 restricted the expansion of CAR-PSCA T cells (2×10⁶ cells-day 0 to 6.1±3.8×10⁷ cells-day 28) in contrast to 4/7R/CAR-PSCA T cells that expanded exponentially (from 2×10⁶ cells to 5.1±3.6×10⁹) and were selectively enriched (increase from 45.9±15% to 86±11% in 4 weeks) (n=3). Further, an additional novel CCR "T-BBR" was generated to provide a costimulatory signal by substituting the endodomain of native TGFβR with that of the co-stimulatory molecule 4-1BB. Transgenic co-expression of T-BBR (39-64% double-positive cells, n=5) was sufficient to protect CAR T cells from the potent inhibitory effects of TGFβ1 (1×10⁶-day 0 to 4.6±3.9×10⁶- day 21-CAR/TBBR; 1×10⁶-day 0 to 2±0.8×10⁵- day 14 - CAR alone). Finally, the selectivity of the cells was compared to 3G.CAR T cell *in vitro* using targets that recapitulate normal (PSCA+ only) vs tumor tissue (PSCA+IL4+TGFβ1+). While 3G.CAR-PSCA T cells killed indiscriminately, the cells were able to selectively eliminate only tumor targets and not "normal" surrogate targets. One can confirm the selectively of SmarT cells *in vivo* using routine methods in the art.

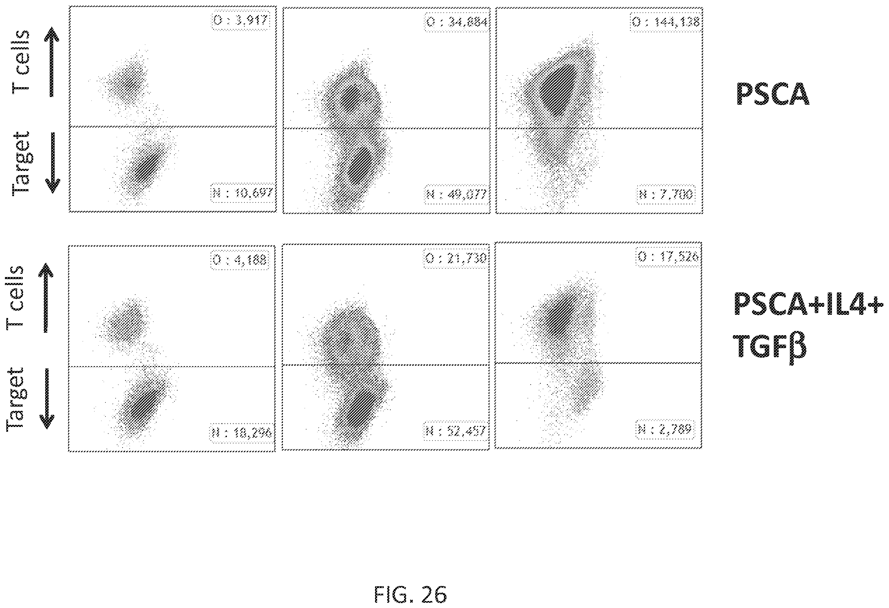

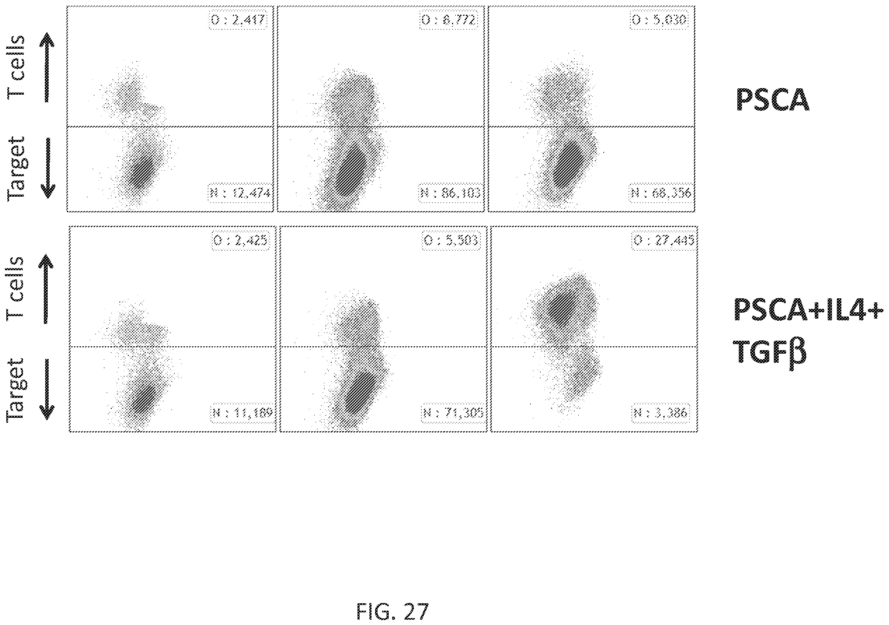

The therapeutic value of the tripartite T cells was demonstrated. Whereas 3G CAR T cells indiscriminately kill PSCA+ targets (FIG. 26), tripartite immune cells (comprising a signal for T cell activation, persistence, and cytokine release) exhibit preferential anti-tumor activity in presence of tumor signature (PSCA+ tumor cells in the presence of II,4 and TGFβ) (FIG. 27).

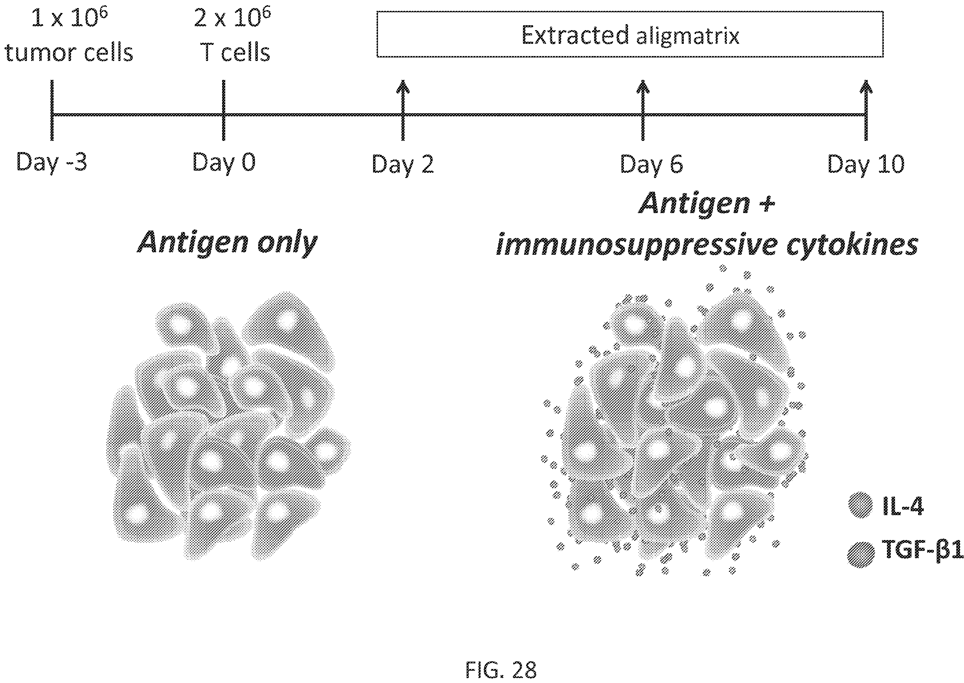

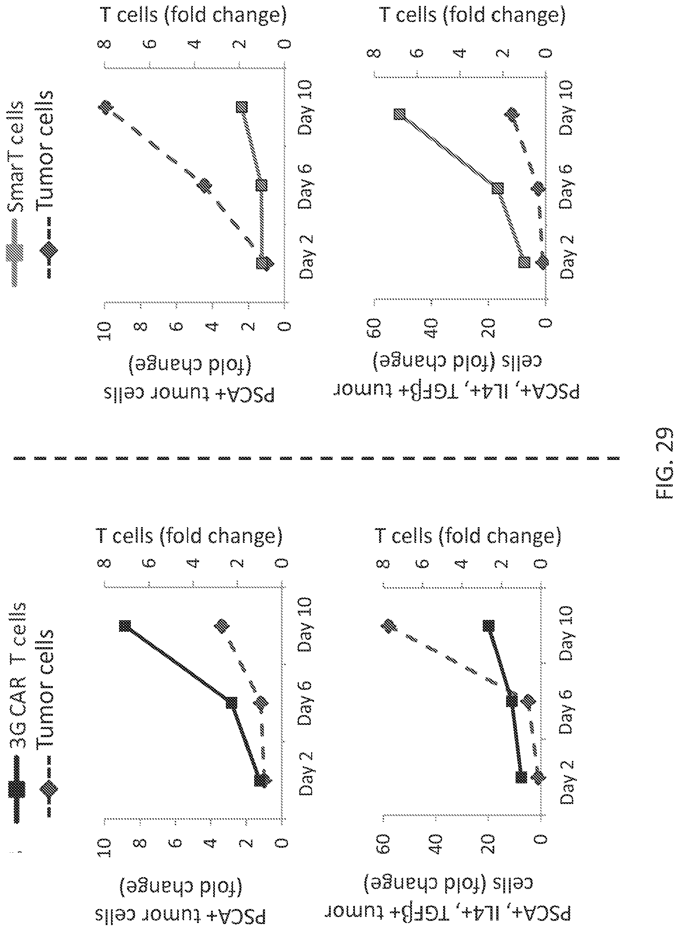

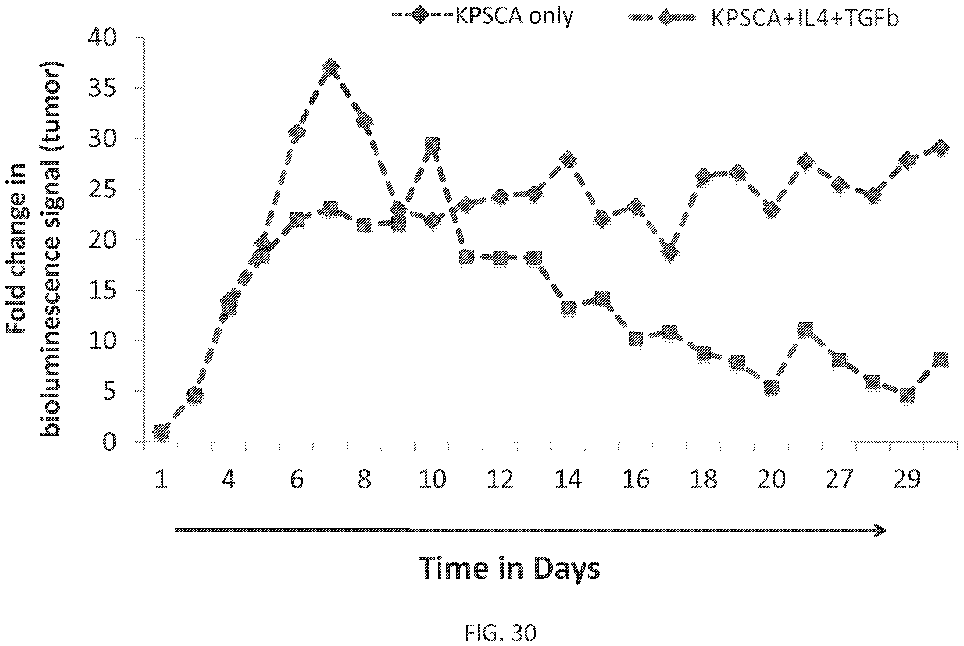

Tripartite immune cells were compared against 3G CAR T cells using a particular culturing system having a gas permeable bottom (G-Rex) (FIG. 28). The tripartite cells exhibited preferential anti-tumor activity against PSCA+, IL4+, TGFβ+ targets (FIG. 29). FIG. 30 shows preferential anti-tumor activity of the tripartite immune cells.

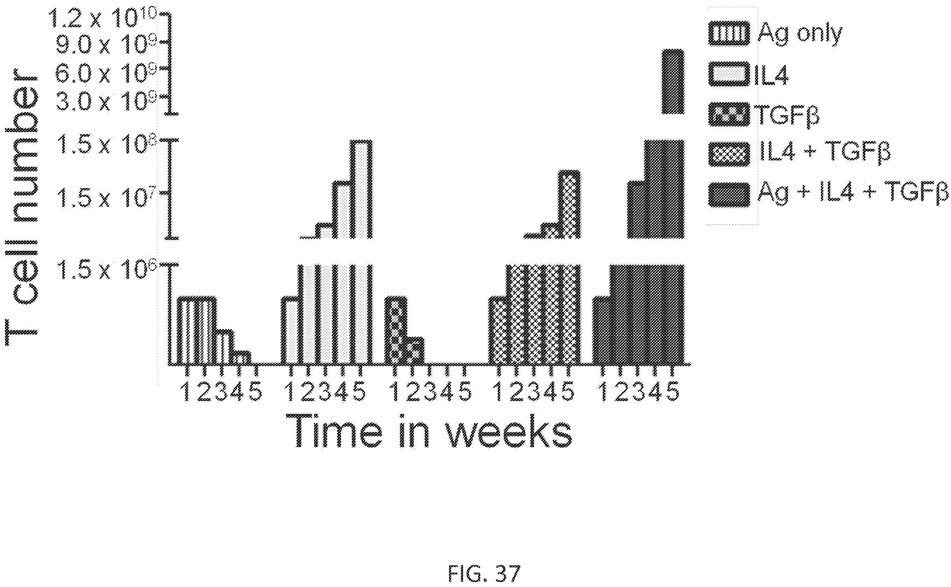

FIG. 37 shows that tripartite T cells exhibit remarkable T cell expansion in presence of the tumor molecular signature that can initiate signals 1, 2 and 3 simultaneously. In contrast, triggering signal 1, 2, or 3 independently results in insufficient T cell response.

Most importantly, whereas 3G CAR T cells indiscriminately kill PSCA-positive targets, the tripartite immune cells exhibit preferential anti-tumor activity against PSCA+, IL4+, TGFβ+ targets.

### REFERENCES

### Patents and Patent Applications

U.S. Patent No. 5,686,281
U.S. Patent No. 5,843,728
U.S. Patent No. 5,906,936
U.S. Patent No. 6,319,494
U.S. Patent No. 7,446,190
U.S. Patent No. 7,741,465
U.S. Patent No. 8,399,645
U.S. Patent Application Publication No. 2012/0148552
WO 94/20627

### Non-Patent Literature

Straathof et al., Blood 105:4247-4254 (2005)
Thomas and Capecchi, Cell. 1987 Nov 6;51(3):503-12
Mansour, et al., Nature. 1988 Nov 24;336(6197):348-52
Joyner, et al., Nature. 1989 Mar 9;338(6211):153-6
Gossen and Bujard (Proc. Natl. Acad. Sci. USA 89 (1992), 5547-5551)
Gossen et al. (Trends Biotech. 12 (1994), 58-62)
Crook (1989) EMBO J. 8, 513-519
Sambrook et al. Molecular Cloning: A Laboratory Manual (1989)
Ausubel, Current Protocols in Molecular Biology, Green Publishing Associates and
Wiley Interscience, N.Y. (1989), (1994)
Reiss, Plant Physiol. (Life-Sci. Adv.) 13 (1994), 143-149
Herrera-Estrella, EMBO J. 2 (1983), 987-995
Marsh, Gene 32 (1984), 481-485)
Hartman, Proc. Natl. Acad. Sci. USA 85 (1988), 8047
McConlogue, 1987, In: Current Communications in Molecular Biology, Cold Spring Harbor Laboratory ed.
Tamura, Biosci. Biotechnol. Biochem. 59 (1995), 2336-2338
Giacomin, Pl. Sci. 116 (1996), 59-72
Scikantha, J. Bact. 178 (1996), 121
Gerdes, FEBS Lett. 389 (1996), 44-47
Jefferson, EMBO J. 6 (1987), 3901-3907

## Claims

1. A composition, comprising an engineered T cell that separately expresses:
1) a chimeric antigen receptor (CAR) that provides antigen signaling for the cell upon binding of PSCA;
2) a co-stimulation receptor that provides co-stimulation for the cell, which is a chimeric cytokine receptor comprising a TGFβR exodomain and a 4-1BB endodomain; and
3) a cytokine receptor that provides cytokine stimulation for the cell, which is a chimeric cytokine receptor comprising an IL-4 receptor exodomain and an IL-7 receptor endodomain;
wherein the antigen receptor and the co-stimulation receptor are different molecules.

2. The composition of claim 1, wherein the TGFβR exodomain is the TGFβRII exodomain.

3. The composition of claim 1, wherein the antigen receptor, the co-stimulation receptor, and the cytokine receptor n are all expressed from the same expression vector or from different expression vectors.

4. The composition of claim 3, wherein the vector is a viral vector or a non-viral vector; optionally wherein the viral vector is a retroviral vector, lentiviral vector, adenoviral vector, or adeno-associated viral vector.

5. The composition of any one of claims 1- 4, wherein the cell further comprises a naturally occurring or engineered T cell receptor that targets a tumor antigen that is:
(i) the same antigen of the composition; or
(ii) a different antigen from the antigen of the composition.

6. The composition of claim 1, wherein the CAR is an artificial receptor that lacks a costimulatory domain.

7. The composition of claim 1, wherein:
(i) the co-stimulation receptor comprises a single endodomain; or
(ii) the cytokine receptor comprises a single endodomain.

8. The composition of any of claims 1-7, for use in a method of treating an individual in need of immunotherapy for a medical condition, comprising the step of delivering a therapeutically effective amount of the composition to the individual.

9. The composition for use according to claim 8, wherein the medical condition is cancer.

10. The composition for use according to claim 9, wherein the cancer has a tumor microenvironment comprising the antigen and soluble factors, the levels of which are sufficient to activate the cells through all of the molecules (1), (2), and (3).

11. A kit comprising the composition of any one of claims 1-7, said composition housed in a suitable container.

## Patentansprüche

1. Zusammensetzung, umfassend eine veränderte T-Zelle, die separat Folgendes exprimiert:
1) einen chimären Antigenrezeptor (CAR), der Antigensignalisierung für die Zelle beim Binden von PSCA bereitstellt;
2) einen Kostimulationsrezeptor, der Kostimulation für die Zelle bereitstellt, der ein chimärer Zytokinrezeptor ist, der eine TGFßR-Exodomäne und eine 4-1BB-Endodomäne umfasst; und
3) einen Zytokinrezeptor, der Zytokinstimulation für die Zelle bereitstellt, der ein chimärer Zytokinrezeptor ist, der eine II,-4-Rezeptor-Exodomäne und eine IL-7-Rezeptor-Endodomäne umfasst;
wobei der Antigenrezeptor und der Kostimulationsrezeptor unterschiedliche Moleküle sind.

2. Zusammensetzung nach Anspruch 1, wobei die TGF(3R-Exodomäne die TGFßRII-Exodomäne ist.

3. Zusammensetzung nach Anspruch 1, wobei der Antigenrezeptor, der Kostimulationsrezeptor und der Zytokinrezeptor n alle aus demselben Expressionsvektor oder aus unterschiedlichen Expressionsvektoren exprimiert sind.

4. Zusammensetzung nach Anspruch 3, wobei der Vektor ein viraler Vektor oder ein nichtviraler Vektor ist; wobei optional der virale Vektor ein retroviraler Vektor, lentiviraler Vektor, adenoviraler Vektor oder Adeno-assoziierter viraler Vektor ist.

5. Zusammensetzung nach einem der Ansprüche 1-4, wobei die Zelle ferner einen natürlich vorkommenden oder veränderten T-Zell-Rezeptor umfasst, der ein Tumorantigen anzielt, das Folgendes ist:
(i) das gleiche Antigen aus der Zusammensetzung; oder
(ii) ein unterschiedliches Antigen als das Antigen aus der Zusammensetzung.

6. Zusammensetzung nach Anspruch 1, wobei der CAR ein künstlicher Rezeptor ist, dem eine kostimulatorische Domäne fehlt.

7. Zusammensetzung nach Anspruch 1, wobei:
(i) der Kostimulationsrezeptor eine einzelne Endodomäne umfasst; oder
(ii) der Zytokinrezeptor eine einzelne Endodomäne umfasst.

8. Zusammensetzung nach einem der Ansprüche 1-7 zur Verwendung in einem Verfahren des Behandelns eines Individuums, das Immuntherapie für eine Erkrankung benötigt, umfassend den Schritt des Zuführens einer therapeutisch wirksamen Menge der Zusammensetzung an das Individuum.

9. Zusammensetzung zur Verwendung nach Anspruch 8, wobei die Erkrankung Krebs ist.

10. Zusammensetzung zur Verwendung nach Anspruch 9, wobei der Krebs eine Tumormikroumgebung hat, die das Antigen und lösliche Faktoren umfasst, deren Menge ausreichend ist, um die Zellen durch alle Moleküle (1), (2) und (3) zu aktivieren.

11. Kit, umfassend die Zusammensetzung nach einem der Ansprüche 1-7, wobei die Zusammensetzung in einem geeigneten Behälter untergebracht ist.

## Revendications

1. Composition comprenant une cellule T modifiée qui exprime séparément :
1) un récepteur d'antigène chimérique (CAR) qui fournit une signalisation antigénique à la cellule lors de la liaison du PSCA ;
2) un récepteur de co-stimulation qui assure la co-stimulation de la cellule, qui est un récepteur de cytokine chimérique comprenant un exodomaine TGFβR et un endodomaine 4-1BB ; et
3) un récepteur de cytokine qui fournit une stimulation de cytokine à la cellule, qui est un récepteur de cytokine chimérique comprenant un exodomaine de récepteur d'IL-4 et un endodomaine de récepteur d'IL-7 ;
le récepteur d'antigène et le récepteur de co-stimulation étant des molécules différentes.

2. Composition selon la revendication 1, dans laquelle l'exodomaine TGFβR est l'exodomaine TGFβRII.

3. Composition selon la revendication 1, dans laquelle le récepteur d'antigène, le récepteur de co-stimulation et le récepteur de cytokine n sont tous exprimés à partir du même vecteur d'expression ou à partir de vecteurs d'expression différents.

4. Composition selon la revendication 3, dans laquelle le vecteur est un vecteur viral ou un vecteur non viral ;
éventuellement, le vecteur viral étant un vecteur rétroviral, un vecteur lentiviral , un vecteur adénoviral ou un vecteur viral adéno-associé.

5. Composition selon l'une quelconque des revendications 1 à 4, dans laquelle la cellule comprend en outre un récepteur de cellule T naturelle ou modifiée qui cible un antigène tumoral qui est :
(i) le même antigène de la composition ; ou
(ii) un antigène différent de l'antigène de la composition.

6. Composition selon la revendication 1, dans laquelle le CAR est un récepteur artificiel dépourvu de domaine de co-stimulation.

7. Composition selon la revendication 1, dans laquelle :
(i) le récepteur de co-stimulation comprend un seul endodomaine ; ou
(ii) le récepteur de cytokine comprend un seul endodomaine.

8. Composition selon l'une quelconque des revendications 1 à 7, à utiliser dans un procédé de traitement d'un individu ayant besoin d'une immunothérapie pour une condition médicale, comprenant l'étape consistant à administrer une quantité thérapeutiquement efficace de la composition à l'individu.

9. Composition à utiliser selon la revendication 8, dans laquelle l'état médical est le cancer.

10. Composition à utiliser selon la revendication 9, dans laquelle le cancer a un microenvironnement tumoral comprenant l'antigène et les facteurs solubles, dont les niveaux sont suffisants pour activer les cellules à travers toutes les molécules (1), (2) et (3)..

11. Kit comprenant la composition selon l'une quelconque des revendications 1 à 7, ladite composition étant logée dans un récipient approprié.
